# FASCICULE DE BREVET EUROPEEN

(11) **EP 2 178 881 B1**
(45) Date de publication et mention de la délivrance du brevet: **02.10.2013**
(21) Numéro de dépôt: 08845285.9
(22) Date de dépôt: 06.08.2008
(51) Int. Cl.: C07D 487/04, A61K 31/5025, A61P 7/00, A61P 25/00, A61P 35/00, A61P 9/00, A61P 11/00, C07D 249/12, C07D 237/20, A61P 19/02, A61P 11/06, A61P 9/10, A61P 7/02, A61P 3/10, A61P 3/00, C07D 487/02, C07D 237/06, C07D 249/10, C07D 235/04, C07D 277/82

(54) **NOUVEAUX DERIVES DE 6-TRIAZOLOPYRIDAZINE-SULFANYL BENZOTHIAZOLE ET BENZIMIDAZOLE, LEUR PROCEDE DE PREPARATION, LEUR APPLICATION A TITRE DE MEDICAMENTS, COMPOSITIONS PHARMACEUTIQUES ET NOUVELLE UTILISATION NOTAMMENT COMME INHIBITEURS DE MET**
NEUE 6-TRIAZOLOPYRIDAZINSULFANYL-BENZOTHIAZOL- UND BENZIMIDAZOL-DERIVATE, VERFAHREN ZU IHRER HERSTELLUNG, IHRE ANWENDUNG ALS MEDIKAMENTE UND PHARMAZEUTISCHE ZUSAMMENSETZUNG SOWIE IHRE NEUE VERWENDUNG ALS MET-HEMMER
NOVEL 6-TRIAZOLOPYRIDAZINESULFANYL BENZOTHIAZOLE AND BENZIMIDAZOLE DERIVATIVES, METHOD FOR PRODUCTION THEREOF AND APPLICATION AS MEDICAMENTS AND PHARMACEUTICAL COMPOSITIONS AND NOVEL USE AS MET INHIBITORS

(30) Priorité: 09.08.2007 FR 0705789; 02.04.2008 FR 0801819
(43) Date de publication de la demande: 28.04.2010
(73) Titulaire: SANOFI, 75008 Paris (FR)
(72) Inventeur: ALBERT, Eva, F-75013 Paris (FR); BACQUE, Eric, F-75013 Paris (FR); NEMECEK, Conception, F-75013 Paris (FR); UGOLINI, Antonio, F-75013 Paris (FR); WENTZLER, Sylvie, F-75013 Paris (FR)
(74) Mandataire: Le Coupanec, Pascale A.M.P.
(86) Numéro de dépôt international: PCT/FR2008/001172
(87) Numéro de publication internationale: WO 2009/056692

(56) Documents cités:
- WO-A-2007/064797
- WO-A-2007/075567
- WO-A-2007/138472
- WO-A-2008/008539
- WO-A-2008/051808
- ANONYMOUS: "CHEMICAL BOOK : 2-amino-6-benzothiazolethiol" [Online] 2007, , XP002523730 Extrait de l'Internet: URL:http://www.chemicalbook.com/ChemicalPr oductProperty_EN_CB81296260.htm> [extrait le 2009-04-15] le document en entier

## Description

La présente invention concerne de nouveaux dérivés de 6-triazolopyridazine-sulfanyl benzothiazole, leur procédé de préparation, les nouveaux intermédiaires obtenus, leur application à titre de médicaments, les compositions pharmaceutiques les renfermant et la nouvelle utilisation de tels dérivés de 6-triazolopyridazine-sulfanyl benzothiazole.

La présente invention concerne plus particulièrement de nouveaux dérivés de 6-triazolopyridazine-sulfanyl benzothiazole présentant une activité anticancéreuse, via la modulation de l'activité de protéines, en particulier des kinases.

A ce jour, la plupart des composés commerciaux utilisés en chimiothérapie sont des cytotoxiques qui posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses, et qui ne seraient pas ou peu exprimés dans les cellules saines. Les protéines kinase sont une famille d'enzymes qui catalysent la phosphorylation de groupes hydroxy de résidus spécifiques de protéines tels que des résidus tyrosine, sérine ou thréonine. De telles phosphorylations peuvent largement modifier la fonction des protéines : ainsi, les protéines kinases jouent un rôle important dans la régulation d'une grande variété de processus cellulaires, incluant notamment le métabolisme, la prolifération cellulaire, l'adhésion et la motilité cellulaire, la différentiation cellulaire ou la survie cellulaire, certaines protéines kinases jouant un rôle central dans l'initiation, le développement et l'achèvement des évènements du cycle cellulaire.

Parmi les différentes fonctions cellulaires dans lesquelles l'activité d'une protéine kinase est impliquée, certains processus représentent des cibles attractives pour traiter certaines maladies. Comme exemple, on peut citer notamment l'angiogénèse et le contrôle du cycle cellulaire et ainsi que celui de la prolifération cellulaire, dans lesquels les protéines kinases peuvent jouer un rôle essentiel. Ces processus sont notamment essentiels pour la croissance des tumeurs solides ainsi que d'autres maladies : notamment des molécules inhibitrices de telles kinases sont susceptibles de limiter des proliférations cellulaires non désirées telles que celles observées dans les cancers, et peuvent intervenir dans la prévention, la régulation ou le traitement de maladies neurodégénératives telles que la maladie d'Alzheimer ou encore l'apoptose neuronale.

WO 2007/064797 décrit des dérivés de triazolopyridazine en tant qu'inhibiteurs de c-Met utiles dans le traitement du cancer.

La présente invention décrit de nouveaux dérivés dotés d'effets inhibiteurs vis-à-vis de protéines kinases. Ces produits peuvent ainsi notamment être utilisés pour la prévention ou le traitement de maladies pouvant être modulées par l'inhibition de protéines kinases.

Les produits selon la présente invention présentent notamment une activité anticancéreuse, via la modulation de l'activité de kinases. Parmi les kinases pour lesquelles une modulation de l'activité est recherchée, MET ainsi que des mutants de la protéine MET sont préférées.

La présente invention concerne également l'utilisation desdits dérivés pour la préparation d'un médicament destiné au traitement de l'homme.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, MET est préférée.

Dans la partie pharmacologique ci-après, il est montré dans des tests biochimiques et sur des lignées cellulaires, que les produits de la présente demande inhibent ainsi notamment l'activité d'autophosphorylation de MET et la prolifération des cellules dont la croissance dépend de MET ou de ses formes mutantes.

MET, ou Hepatocyte Growth Factor Receptor, est un récepteur à activité tyrosine kinase exprimé en particulier par les cellules épithéliales et endothéliales. Le HGF, Hepatocyte Growth Factor, est décrit comme le ligand spécifique de MET. Le HGF est sécrété par les cellules mésenchymales et active le récepteur MET qui s'homodimérise. Par voie de conséquence, le récepteur s'autophosphoryle sur les tyrosines du domaine catalytique Y1230, Y1234 et Y1235.

La stimulation de MET par le HGF induit la prolifération, le scattering (ou dispersion), la motilité cellulaire, la résistance à l'apoptose, l'invasion et l'angiogénèse.

MET de même que le HGF, sont retrouvés surexprimés dans de nombreuses tumeurs humaines et une grande variété de cancers. MET est aussi retrouvé amplifié dans des tumeurs gastriques et glioblastomes. De nombreuses mutations ponctuelles du gène MET ont aussi été décrites dans des tumeurs, en particulier dans le domaine kinase mais aussi dans le domaine juxtamembranaire et le SEMA domain. Surexpression, amplification ou mutations provoquent l'activation constitutive du récepteur et une dérégulation de ses fonctions.

La présente invention concerne ainsi notamment de nouveaux inhibiteurs de la protéine kinase MET et de ses mutants, pouvant être utilisés pour un traitement anti-prolifératif et anti-métastatique notamment en oncologie.

La présente invention concerne également de nouveaux inhibiteurs de la protéine kinase MET et de ses mutants, pouvant être utilisés pour un traitement anti- angiogénique, notamment en oncologie.

La présente invention a pour objet les produits de formule (I"b) : 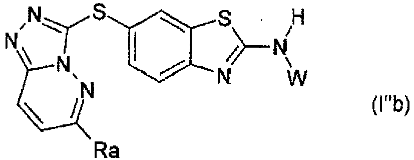 dans laquelle
- Ra représente un atome d'hydrogène ou bien un atome de chlore ou bien le radical : 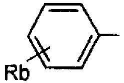 avec Rb représente un atome d'halogène ou un radical S-hétéroaryle éventuellement substitué par un radical choisi parmi les atomes d'halogène et les radicaux hydroxyle, alkyle et alcoxy renfermant de 1 à 4 atomes de carbone, NH2, NHalk et N(alk)2 ;
   W représente un atome d'hydrogène ou un radical alkyle ou le radical COR dans lequel R représente :
   - un radical alkyle éventuellement substitué par OCH3 ou NR3R4 ;
   - un radical cycloalkyle
   - un radical alcoxy éventuellement substitué par OCH3 ou NR3R4, càd un radical O-(CH2)n- OCH3 ou un radical O-(CH2)n-NR3R4 ; un radical O-phényle ou O-(CH2)n-phényle, avec n représente un entier de 1 à 2 :
- ou le radical NR1R2, dans lequel R1 et R2 sont tels que l'un de R1 et R2 représente un atome d'hydrogène, un radical cycloalkyle ou un radical alkyle et l'autre de R1 et R2 représente un radical alkyle éventuellement substitué par NR3R4, ou bien R1 et R2 forment avec l'atome d'azote auquel ils sont liés un radical cyclique renfermant de 3 à 10 chaînons et renfermant éventuellement un ou plusieurs autres hétéroatomes choisi(s) parmi O, S, N et NH ;
avec NR3R4 tel que R3 et R4, identiques ou différents, représentent un atome d'hydrogène ou un radical alkyle ou bien R3 et R4 forment avec l'atome d'azote auquel ils sont liés un radical cyclique renfermant éventuellement un ou plusieurs autres hétéroatomes choisi(s) parmi O, S, N et NH, ce radical y compris l'éventuel NH qu'il contient étant éventuellement substitué par un ou plusieurs radicaux choisis parmi les radicaux alkyle ;
avec le terme radical alkyle ou Alk désignant un radical alkyle renfermant de 1 à 6 atomes de carbone,
avec le terme radical cycloalkyle désignant un radical carbocyclique saturé renfermant 3 à 10 atomes de carbone,
avec le terme alcoxy désignant un radical renfermant de 1 à 4 atomes de carbone,
avec le terme hétéroaryle désignant un radical monocyclique ou bicyclique renfermant de 5 à 12 chaînons,
lesdits produits de formule (I"b) étant sous toutes les formes isomères possibles racémiques, énantiomères et diastéréoisomères, ainsi que les sels d'addition avec les acides minéraux et organiques ou avec les bases minérales et organiques desdits produits de formule (I"b).

Dans les produits de formule (I) et dans ce qui suit :
- le terme radical alkyle (ou Alk) désigne les radicaux, linéaires et le cas échéant ramifiés, méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : on préfère les radicaux alkyles renfermant de 1 à 6 atomes de carbone et plus particulièrement les radicaux alkyle renfermant de 1 à 4 atomes de carbone de la liste ci-dessus ;
- le terme radical alcoxy désigne les radicaux linéaires et le cas échéant ramifiés, méthoxy, éthoxy, propoxy, isopropoxy, butoxy linéaire, secondaire ou tertiaire, pentoxy ou hexoxy ainsi que leurs isomères de position linéaires ou ramifiées : on préfère les radicaux alkoxy renfermant de 1 à 4 atomes de carbone de la liste ci-dessus ;
- le terme atome d'halogène désigne les atomes de chlore, de brome, d'iode ou de fluor et de préférence l'atome de chlore, de brome ou de fluor.
- le terme radical cycloalkyle désigne un radical carbocyclique saturé renfermant 3 à 10 atomes de carbone et désigne ainsi notamment les radicaux cyclopropyle, cyclobutyle, cyclopentyle et cyclohexyle et tout particulièrement les radicaux cyclopropyl, cyclopentyle et cyclohexyle ;
- le terme radical hétérocycloalkyle désigne ainsi un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre : on peut citer par exemple les radicaux morpholinyle, thiomorpholinyle, aziridyle, azétidyle, pipérazinyle, pipéridyle, homopipérazinyle, pyrrolidinyle, imidazolidinyle, pyrazolidinyle, tétrahydrofuryle, tétrahydrothiényle, hexahydropyranne, oxodihydropyridazinyle, ou encore oxétanyle tous ces radicaux étant éventuellement substitués ;
- les termes aryle et hétéroaryle désignent des radicaux insaturés ou partiellement insaturés, respectivement carbocycliques et hétérocycliques, monocycliques ou bicycliques, renfermant au plus 12 chaînons, pouvant éventuellement contenir un chaînon -C(O), les radicaux hétérocycliques contenant un ou plusieurs hétéroatomes identiques ou différents choisis parmi O, N, ou S avec N, le cas échéant, éventuellement substitué ;
- le terme radical aryle désigne ainsi des radicaux monocycliques ou bicycliques renfermant 6 à 12 chaînons tels que par exemple les radicaux phényle, naphtyle, biphényle, indényle, fluorényle et anthracényle, plus particulièrement les radicaux phényle et naphtyle et encore plus particulièrement le radical phényle. On peut noter qu'un radical carbocyclique contenant un chaînon -C(O) est par exemple le radical tétralone ;
- le terme radical hétéroaryle désigne ainsi des radicaux monocycliques ou bicycliques renfermant 5 à 12 chaînons : des radicaux hétéroaryles monocycliques tels que par exemple les radicaux thiényle tel que 2-thiényle et 3-thiényle, furyle tel que 2-furyle, 3-furyle, pyrannyle, pyrrolyle, pyrrolinyle, pyrazolinyle, imidazolyle, pyrazolyle, pyridyle tel que 2-pyridyle, 3-pyridyle et 4-pyridyle, pyrazinyle, pyrimidinyle, pyridazinyle, oxazolyle, thiazolyle, isothiazolyle, diazolyle, thiadiazolyle, thiatriazolyle, oxadiazolyle, isoxazolyle tel que 3- ou 4-isoxazolyle, furazannyle, tétrazolyle libre ou salifié, tous ces radicaux étant éventuellement substitués parmi lesquels plus particulièrement les radicaux thiényle tel que 2-thiényle et 3-thiényle, furyle tel que 2-furyle, pyrrolyle, pyrrolinyle, pyrazolinyle, imidazolyle, pyrazolyle, oxazolyle, isoxazolyle, pyridyle, pyridazinyle, ces radicaux étant éventuellement substitués; des radicaux hétéroaryles bicycliques tels que par exemple les radicaux benzothiényle tel que 3-benzothiényle, benzothiazolyle, quinolyle, isoquinolyle, dihydroquinolyle, quinolone, tétralone, adamentyl, benzofuryle, isobenzofuryle, dihydrobenzofuranne, éthylènedioxyphényle, thianthrényle, benzopyrrolyle, benzimidazolyle, benzoxazolyle, thionaphtyle, indolyle, azaindolyle, indazolyle, purinyle, thiénopyrazolyle, tétrahydroindazolyle, tétrahydrocyclopentapyrazolyle, dihydrofuropyrazolyle, tétrahydropyrrolopyrazolyle, oxotétrahydropyrrolopyrazolyle, tétrahydropyranopyrazolyle, tétrahydropyridinopyrazolyle ou oxodihydropyridino-pyrazolyle, tous ces radicaux étant éventuellement substitués ;

Comme exemples de radicaux hétéroaryles ou bicycliques, on peut citer plus particulièrement les radicaux pyrimidinyle, pyridyle, pyrrolyle, azaindolyle, indazolyle ou pyrazolyle, éventuellement substitués par un ou plusieurs substituants identiques ou différents comme indiqué ci-dessus.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposées différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

Les radicaux cycliques que peuvent former d'une part R1 et R2 avec l'atome d'azote auquel ils sont liés et d'autre part R3 et R4 avec l'atome d'azote auquel ils sont liés, sont éventuellement substitués par un ou plusieurs radicaux choisis parmi ceux indiqués ci-dessus pour les éventuels substituants des radicaux hétérocycloalkyle càd un ou plusieurs radicaux choisis parmi les atomes d'halogène, les radicaux hydroxyle, oxo, alcoxy, NH2; NHalk, N(alk)2, et les radicaux alkyle, hétérocycloalkyle, CH2-hétérocycloalkyle, phényle, CH2-phényle, hétéroaryle, et CO-phényle, tels que dans ces derniers radicaux les radicaux alkyle, hétérocycloalkyle et phényle sont eux-mêmes éventuellement substitués par un ou plusieurs radicaux choisis parmi les atomes d'halogène et les radicaux hydroxyle, oxo, alkyle et alcoxy renfermant de 1 à 4 atomes de carbone, NH2; NHalk et N(alk)2,

Les radicaux cycliques que peuvent former d'une part R1 et R2 avec l'atome d'azote auquel ils sont liés et d'autre part R3 et R4 avec l'atome d'azote auquel ils sont liés, sont notamment éventuellement substitués par un ou plusieurs radicaux identiques ou différents choisis parmi les atomes d'halogène et les radicaux alkyle, hydroxyle, alcoxy, CH2-pyrrolidinyle, CH2-phényle, hétéroaryle, et phényle, dans lesquels les radicaux alkyle, pyrrolidinyle et phényle sont eux-mêmes éventuellement substitués par un ou plusieurs radicaux identiques ou différents choisis parmi les atomes d'halogène et les radicaux alkyle, hydroxyle, oxo et alcoxy.

Les radicaux hétérocycloalkyle tels que définis ci-dessus représentent notamment les radicaux azépanyle, morpholinyle et pyrrolidinyle, pipéridyle, et pipérazinyle eux-mêmes éventuellement substitués, tels que définis ci-dessus ou ci-après.

Lorsque NR1R2 ou NR3R4 forme un cycle comme défini ci-dessus, un tel cycle aminé peut être choisi notamment parmi les radicaux pyrrolidinyle, pyrazolidinyle, pyrazolinyle, pipéridyle, azépinyle, morpholino ou pipérazinyle, ces radicaux étant eux-mêmes éventuellement substitués comme indiqué ci-dessus ou ci-après : par exemple par un ou plusieurs radicaux identiques ou différents choisis parmi les atomes d'halogène et les radicaux alkyle, hydroxyle, alcoxy, phényle et CH2-phényle, les radicaux alkyle ou phényle étant eux-mêmes éventuellement substitués par un ou plusieurs radicaux identiques ou différents choisis parmi les atomes d'halogène et les radicaux alkyle, hydroxyle et alcoxy.

Lorsque dans les produits de formule (I), Ra représente le radical : 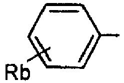 Rb est notamment en position para.

Lorsque Rb défini ci-dessus représente un atome d'halogène, Rb représente notamment fluor.

La présente invention a tout particulièrement pour objet les produits de formule (I"b) tels que définis ci-dessus répondant aux formules suivantes :
- (6-{[6-(4-fluorophényl)[1,2,4]triazolo[4,3-b]pyridazin-3-yl]sulfanyl}-1,3-benzothiazol-2-yl)carbamate de méthyle
- (6-{[6-(4-fluorophényl)[1,2,4]triazolo[4,3-b]pyridazin-3-yl]sulfanyl}-1,3-benzothiazol-2-yl)carbamate de 1,1-diméthyléthyle
- 6-{[6-(4-fluorophényl)[1,2,4]triazolo[4,3-b]pyridazin-3-yl]sulfanyl}-1,3-benzothiazol-2-amine
- 1-(2-morpholin-4-yléthyl)-3-[6-([1,2,4]triazolo[4,3-b]pyridazin-3-ylsulfanyl)-1,3-benzothiazol-2-yl]urée
- 1-(6-{[6-(4-fluorophényl)[1,2,4]triazolo[4,3-b]pyridazin-3-yl]sulfanyl}-1,3-benzothiazol-2-yl)-3-(2-morpholin-4-yléthyl)urée
- 1-{2-[(2R,6S)-2,6-diméthylmorpholin-4-yl]éthyl}-3-(6-{[6-(4-fluorophényl)[1,2,4]triazolo[4,3-b]pyridazin-3-yl]sulfanyl}-1,3-benzothiazol-2-yl)urée
- (6-{[6-(4-fluorophényl)[1,2,4]triazolo[4,3-b]pyridazin-3-yl]sulfanyl}-1,3-benzothiazol-2-yl)carbamate de 2-morpholin-4-yléthyle
- 1-(6-{[6-(4-fluorophényl)[1,2,4]triazolo[4,3-b]pyridazin-3-yl]sulfanyl}-1,3-benzothiazol-2-yl)-3-[2-(4-méthylpipérazin-1-yl)éthyl]urée
- N-(6-{[6-(4-fluorophényl)[1,2,4]triazolo[4,3-b]pyridazin-3-yl]sulfanyl}-1,3-benzothiazol-2-yl)-4-morpholin-4-ylbutanamide
- 1-[2-(diéthylamino)éthy]-3-(6-{[6-(4-fluorophényl)[1,2,4]triazolo[4,3-b]pyridazin-3-yl]sulfanyl}-1,3-benzothiazol-2-yl)urée
- 6-{[6-(4-fluorophényl)[1,2,4]triazolo[4,3-b]pyridazin-3-yl]sulfanyl}-1,3-benzothiazol-2-yl)carbamate de phényle
- N-(6-{[6-(4-fluorophényl)[1,2,4]triazolo[4,3-b]pyridazin-3-yl]sulfanyl}-1,3-benzothiazol-2-yl)acétamide
- 6-{[6-(3-fluorophényl)[1,2,4]triazolo[4,3-b]pyridazin-3-yl]sulfanyl}-1,3-benzothiazol-2-amine
- N-(6-{[6-(4-fluorophényl)[1,2,4]triazolo[4,3-b]pyridazin-3-yl]sulfanyl)-1,3-benzothiazol-2-yl)morpholine-4-carboxamide
- 6-{[6-(2-fluorophényl)[1,2,4]triazolo[4,3-b]pyridazin-3-yl]sulfanyl}-1,3-benzothiazol-2-amine
- N-(6-{[6-(4-fluorophényl)[1,2,4]triazolo[4,3-b]pyridazin-3-yl]sulfanyl}-1,3-benzothiazol-2-yl)cyclopropanecarboxamide
- 1-(6-{[6-(3-fluorophényl)[1,2,4]triazolo[4,3-b]pyridazin-3-yl]sulfanyl}-1,3-benzothiazol-2-yl)-3-[2-(morpholin-4-yl)éthyl]urée
- N-(6-{[6-(3-fluorophényl)[1,2,4]triazolo[4,3-b]pyridazin-3-yl]sulfanyl}-1,3-benzoth iazol-2-yl)acétam ide;
- N-(6-{[6-(4-fluorophényl)[1,2,4]triazolo[4,3-b]pyridazin-3-yl]sulfanyl}-1,3-benzothiazol-2-yl}-2-méthylpropanamide;
- N-(6-{[6-(3-fluorophényl)[1,2,4]triazolo[4,3-b]pyridazin-3-yl]sulfanyl}-1,3-benzothiazol-2-yl)cyclopropanecarboxamide ;
ainsi que les sels d'addition avec les acides minéraux et organiques ou avec les bases minérales et organiques desdits produits de formule (I"b).

La présente invention a encore pour objet tout procédé de préparation des produits de formule (I"b) tels que définis ci-dessus

Les produits selon l'invention peuvent être préparés à partir de méthodes conventionnelles de chimie organique. Les schémas 2, 3, 4, 5, 6 et 7 ci-dessous sont illustratifs des méthodes utilisées pour la préparation des produits de formule (I"b). A ce titre, il ne saurait constituer une limitation de la portée de l'invention, en ce qui concerne les méthodes de préparation des composés revendiqués.

Les produits de formule (I) tels que définis ci-dessus selon la présente invention peuvent ainsi notamment être préparés selon les procédés décrits dans les schémas 2, 3, 4, 5, 6 et 7 ci-dessous.

La présente invention a ainsi également pour objet le procédé de préparation de produits de formule (I"b) selon le schéma 2 tel que défini ci-après.

La présente invention a ainsi également pour objet le procédé de préparation de produits de formule (I"b) selon le schéma 4 tel que défini ci-après.

La présente invention a ainsi également pour objet le procédé de préparation de produits de formule (I"b) selon le schéma 5 tel que défini ci-après.

La présente invention a ainsi également pour objet le procédé de préparation de produits de formule (I"b) selon le schéma 5bis tel que défini ci-après.

La présente invention a ainsi également pour objet le procédé de préparation de produits de formule (I"b) selon le schéma 6 tel que défini ci-après.

La présente invention a ainsi également pour objet le procédé de préparation de produits de formule (I"b) selon le schéma 7 tel que défini ci-après.

Dans l'ensenble des schémas ci-après, dans les formules (I") et (I'), A est S et W et Ra ont les significations indiquées précédemment.

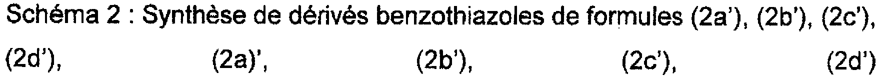

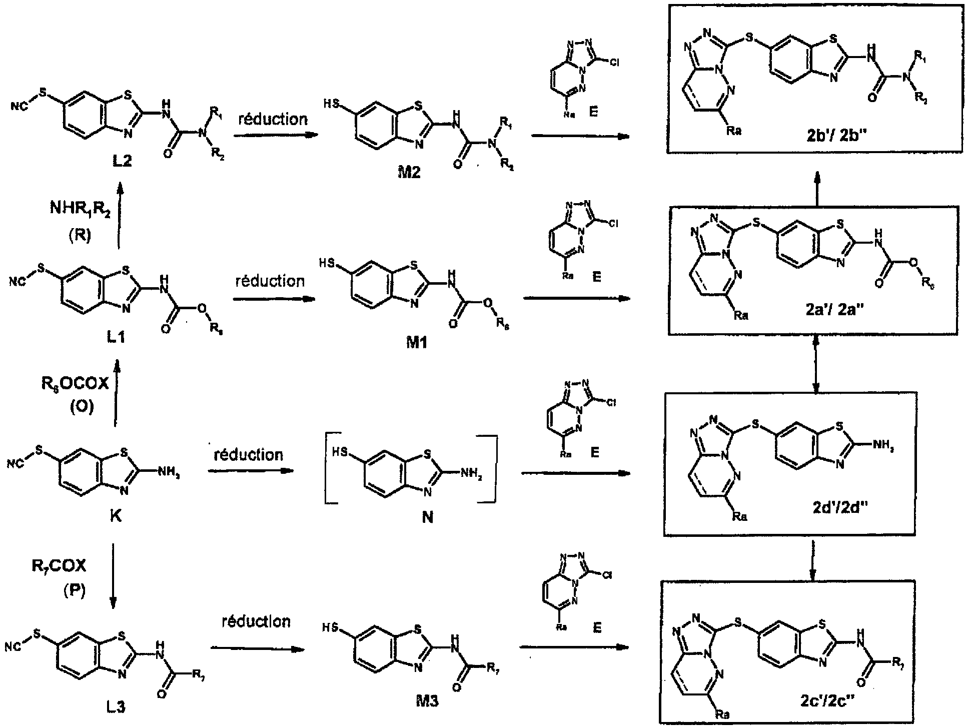

Dans le schéma 2 ci-dessus, les substituants, Ra, R1 et R2 ont les significations indiquées ci-dessus pour les produits de formule (I"b) et le substituant R6, dans les composés de formules (O), (L1), (M1) et (2a')/(2a"), représente un radical alkyle éventuellement substitué par un groupe NR3R4 ou OCH₃ un radical phényle, -(CH2)n-phényle, et n représente un entier de 1 à 2, tel que OR6 représentent les valeurs correspondantes de R tel que défini ci-dessus pour les produits de formule (I"b). Le substituant R7 dans les composés de formules (M3), (L3), (P) et (2c')/(2c") représente un radical cycloalkyle ou alkyle éventuellement substitué par un radical NR3R4 ou OCH₃, dans lesquels R3 et R4 ont les significations indiquées précédemment et n représente un entier de 1 à 2.

dans les composés de formules 2a", 2b", 2c" et 2d", ---- représente une double liaison.

Dans le schéma ci-dessus 2, les benzothiazoles de formule générale (2a"), (2b"), (2c") et (2d") ainsi que leurs analogues réduits de formule générale (2a'), (2b'), (2c') et (2d') peuvent être préparés à partir du thiocyanate de 2-amino-1,3-benzothiazol-6-yle (K) (composé commercial).

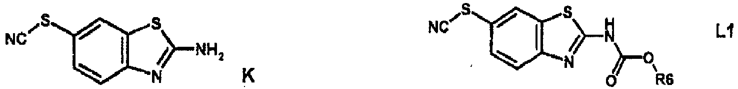

Les carbamates de formule générale (L1) peuvent être obtenus, par exemple, par réaction avec un chlorocarbonate de formule (O) (X=Cl) sur le thiocyanate de 2-amino-1,3-benzothiazol-6-yle (K), dans un solvant tel le tétrahydrofurane, en présence d'une base telle l'hydrogénocarbonate de sodium, à une température voisine de 20°C.

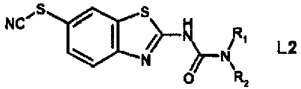

Les composés de formule générale (L2) peuvent être obtenus, par exemple, par réaction des carbamates de formule (L1) où R6=phényl, avec des amines NHR1R2 de formule (R) (avec R1 et R2 tels que définis ci-dessus), en présence d'un solvant aprotique tel le tétrahydrofurane, à une température voisine de 20°C.

Les urées (2b') et (2b") peuvent être obtenues, par exemple, respectivement à partir des carbamates (2a') et (2a") où R6=phényl, de la même manière que les urées (L2) sont obtenues par réaction d'amines sur les carbamates de type (L1).

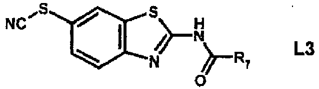

Les composés de formule générale (L3) peuvent être obtenus par exemple :
- par réaction d'un chlorure d'acide de formule (P) (X = Cl) sur le thiocyanate de 2-amino-1,3-benzothiazol-6-yle (K), en présence, par exemple, d'un solvant tel la pyridine, à une température voisine de 20°C.
- par réaction d'un anhydride d'acide de formule (P) (X= OCOR7) sur le thiocyanate de 2-amino-1,3-benzothiazol-6-yle (K), en présence, par exemple, d'un solvant tel la pyridine à une température voisine de 20°C.
- par couplage du thiocyanate de 2-amino-1,3-benzothiazol-6-yle (K) avec un acide de formule (P) (X=OH) dans les conditions, par exemple, décrites par D.D. DesMarteau; V. Montanari (Chem Lett, 2000 (9),1052), en présence de 1-hydroxybenzotriazole et de 1-(3-diméthylaminopropyl)-3-éthylcarbodiimide et en présence d'une base telle la triéthylamine, à une température voisine de 40°C.

De la même manière que les carboxamides (L3) peuvent être obtenues par acylation de l'amine (K), les carboxamides (2c') et (2c") peuvent être obtenues respectivement à partir des amines (2d') et (2d").

Les composés de formule générale (M1), (M2) et (M3) peuvent être obtenus, par exemple, par réduction de composés de formule générale (L1), (L2), (L3) avec du DL-dithiotréitol, en présence de dihydrogénocarbonate de sodium, dans un solvant tel l'éthanol et à une température voisine de 80°C.

Le composé de formule générale (N) peut être préparé in situ par réduction du composé de formule (K) pour donner directement les dérivés amino de formule (2d') et (2d"), par exemple avec du borohydrure de sodium dans un solvant tel la N, N-diméthylformamide, en présence d'une base telle la triéthylamine et à une température voisine de 95°C ou comprise entre 20°C et 95°C.

Plus particulièrement, les benzothiazoles de formule générale (2d') et (2d") peuvent être également préparés respectivement à partir de carbamates de formule (2a') et (2a') où R6 = t-butyl par réaction, par exemple, avec de l'acide trifluoro acétique dans un solvant tel le dichlorométhane, à une température voisine de 20°C.

Réciproquement, les benzothiazoles de formule générale (2a') et (2a") peuvent être également préparés à partir de benzothiazoles de formule respectivement (2d') et (2d"), par exemple, par réaction avec un chlorocarbonate de formule (O) (X=Cl), dans un solvant tel le tétrahydrofurane, en présence d'une base telle l'hydrogénocarbonate de sodium, à une température voisine de 20°C.

Plus particulièrement, les benzothiazoles de formule générale (2a"), (2b"), (2c") et (2d") ainsi que leurs analogues réduits de formule générale (2a'), (2b'), (2c') et (2d') peuvent être préparés par exemple :
1) soit par couplage d'un composé de formule (E) avec des dérivés (M1), (M2) et (M3) et (N) engendré *in situ* par réduction des dérivés (L1), (L2), (L3) et (K) par le borohydrure de sodium, dans un solvant tel la N, N-diméthylformamide et en présence d'une base telle la triéthylamine, à une température voisine de 95°C ou bien comprise entre 50°C et 95°C.
2) soit par couplage des dérivés (M1), (M2) et (M3) isolés et d'un composé de formule (E), en présence de borohydrure de sodium dans un solvant tel la N, N-diméthylformamide et en présence d'une base telle la triéthylamine, à une température voisine de 95°C.
3) soit par couplage des dérivés isolés (M1), (M2) et (M3) et d'un composé de formule (E) dans les conditions, par exemple, décrites par U. Schopfer et coll (Tetrahedron, 2001, 57, 3069) en présence de n-tributyl phosphine, de tert-butylate de potassium, de tris(dibenzylideneacetone)dipalladium (0) et de bis(2-diphénylphosphinophényl)ether dans un solvant tel le toluène à une température voisine de 110°C
4) soit par couplage d'un composé de formule (E) avec des dérivés (M1), (M2) et (M3) et (N) engendré *in situ* par réduction des dérivés (L1), (L2), (L3) et (K) en présence de DL-dithiotréitol et de dihydrogénocarbonate de sodium, dans un solvant tel l'éthanol et à une température voisine de 80°C.

Les conditions réductrices 1) et 2) peuvent donner des produits de formule (2a), (2b), (2c) et (2d) tels que ---- représentent une liaison simple ou double alors que les conditions 3) et 4) donnent des produits de formule (2a), (2b), (2c) et (2d) tels que ---- représentent une liaison double..

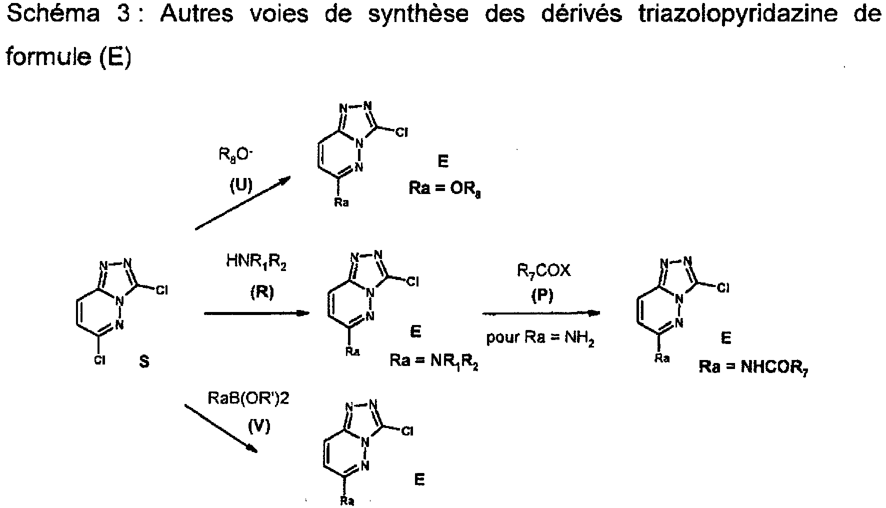

Dans le schéma 3 ci-dessus, les substituants Ra, R1 et R2 ont les significations indiquées ci-dessus pour les produits de formule (I') et (I"). Le substituant R7 représente un radical alkyle ou cycloalkyle.

Le substituant R8 représente :
- soit un radical alkyle éventuellement substitué par un atome de chlore, un radicale hydroxyle ou un radicale hétérocycloalkyle lui-même éventuellement substitué,
- soit un radical cyclolkyle

Les composés de formule (E) peuvent être obtenus, par exemple, comme indiqué dans le schéma 3 ci-dessus, à partir de la 3,6-dichloro[1,2,4]triazolo[4,3-b]pyridazine commerciale de formule (S).

Plus particulièrement, les composés de formule (E) où Ra représente un radical OR8 peuvent être obtenus par le traitement de la 3,6-dichloro[1,2,4]triazolo[4,3-b]pyridazine (S) avec un alcoolate de formule (U), à une température voisine de 80°C et dans un solvant tel la N,N-diméthylformamide.

Plus particulièrement, les composés de formule (E) où Ra représente un radical NR₁R₂ peuvent être obtenus par le traitement de la 3,6-dichloro[1,2,4]triazolo[4,3-b]pyridazine (S) avec une amine de formule (R), à une température voisine de 20°C et dans un solvant tel la N,N-diméthylformamide ou, dans le cas NR₁R₂ est NH2, avec de l'ammoniaque aqueux, dans un solvant tel le dioxanne, dans un tube scellé, à une température comprise entre 70°C et 90°C.

Plus particulièrement, les composés de formule (E) où Ra représente un radical NHCOR7 peuvent être obtenus par réaction d'un composé de formule générale (E) avec Ra = NH2 avec un composé de formule (P) comme décrit pour les composés de formule générale (L3), (1 e') et (1e").

Plus particulièrement, les composés de formule (E) où Ra représente un radical aryle ou hétéroaryle peuvent être obtenus, par exemple :
- à partir des acides boroniques de formule (V) (R'=H), en présence d'hydroxyde de barium octahydrate et de (1,1'-bis (diphénylphosphino)ferrocène) di-chloropalladium (II) dans un solvant tel, par exemple, la N,N-diméthyleformamide, à une température voisine de 80°C.
- ou alternativement, à partir des esters boroniques de formule (V), en présence de dichloro bis (triphénylphosphine) de palladium dans un solvant tel, par exemple, le 1,2-diméthoxyéthane, en présence d'une base telle la soude 1 N, à une température voisine de 80°C.

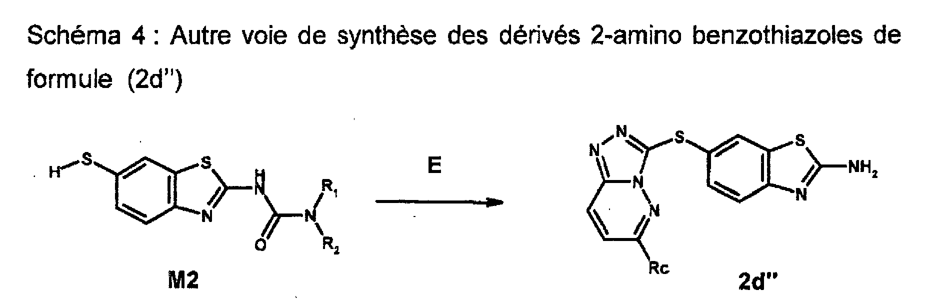

Selon le schéma 4 ci-dessus, les 2-amino benzothiazoles de formule générale (2d") peuvent également être préparés à partir des composés de formule (M2) et un composé de formule (E), en présence de carbonate de potassium, dans un solvant tel le dimethylsulfoxyde. La réaction est effectuée, par exemple, sous microondes, pendant environ 10min, à une température voisine de 190°C. Dans le composé obtenu de formule (2d"), le substituant Rc représente un atome d'hydrogène ou bien un radical hétéroaryle non lié par un atome d'azote ou bien un radical phényle, ces radicaux étant éventuellement substitués comme indiqué ci-dessus pour le substituant Ra.

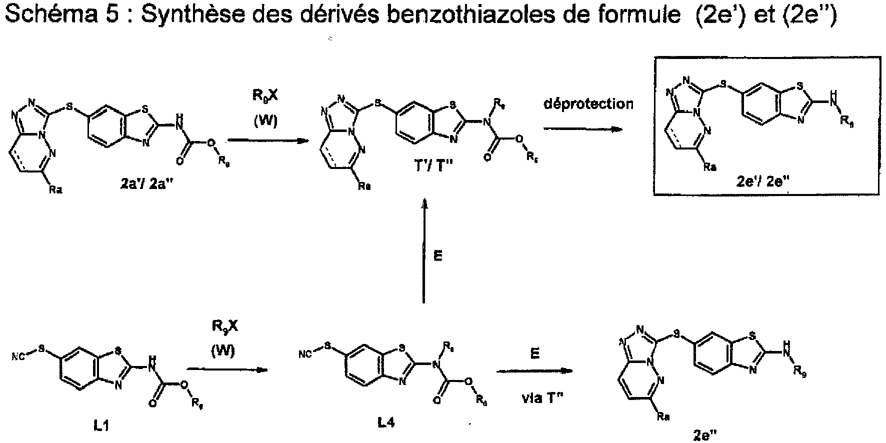

Selon le schéma 5 ci-dessus, les benzothiazoles de formule générale (2e') et (2e") peuvent être préparés respectivement à partir des composés de formule (2a') et (2a").

Dans le schéma 5 ci-dessus, le substituant OR6 représente préférentiellement O-t-Butyl. Le substituant R9 représente un radical alkyle ou cycloalkyle éventuellement substitué par un radical alcoxy, hétérocycloalkyle ou NR3R4 (R3 et R4 tels que définis ci-dessus).

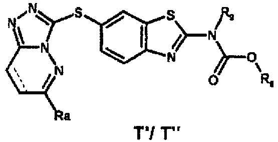

Les carbamates de formule générale (T') et (T") peuvent être obtenus respectivement par réaction de carbamates de formule générale (2a') et (2a") avec R6=tBu préférentiellement, par exemple, avec des halogénures d'alkyle de formule (W), dans un solvant tel la N,N-diméthylformamide, en présence d'hydrure de sodium, à une température comprise entre 20 et 90°C.

Les benzothiazoles de formule générale (2e') et (2e") peuvent également être préparés à partir des composés de formule (L1), avec de préférence R6=tBu, via les composés de formule (T') et (T").

Plus particulièrement, les composés de formule générale (2e') et (2e") peuvent être obtenus respectivement par traitement des composés (T') et (T") isolés, par exemple, avec de l'acide trifluoroacétique, dans un solvant tel le dichlorométhane, à une température voisine de 20°C,

Alternativement, les composés de formule générale (2e") peuvent être obtenus directement par réaction des composés de formule (L4) et (E), via le composé (T") formé *in situ*, par exemple, en présence de DL-dithiotréitol et de dihydrogénocarbonate de sodium, dans un solvant tel l'éthanol et à une température voisine de 80°C, suivi éventuellement d'un traitement *in situ* à l'acide trifluoroacétique à 20°C si nécessaire.

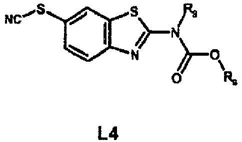

Les carbamates de formule générale (L4) peuvent être obtenus par réaction de carbamates de formule générale (L1), par exemple, avec des halogénures d'alkyle de formule (W), dans un solvant tel la N,N-diméthylformamide, en présence d'hydrure de sodium, à une température comprise entre 20 et 90°C.

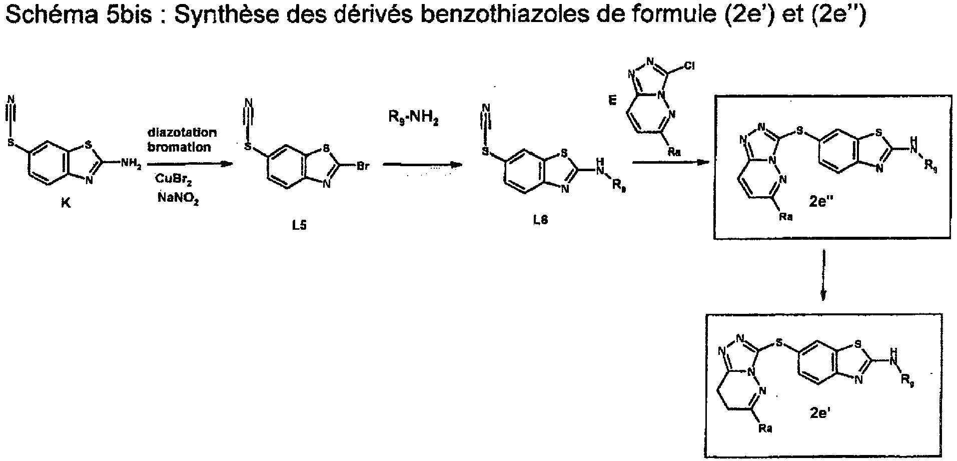

Alternativement, selon le schéma 5bis ci-dessus, les benzothiazoles de formule générale (2e") peuvent être préparés à partir des composés de formules (L6) et (E), par exemple, en présence de DL-dithiotréitol et de dihydrogénocarbonate de sodium, dans un solvant tel l'éthanol et à une température voisine de 80°C.

Les benzothiazoles de formule générale (2e') peuvent être préparés à partir des composés de formule (2e"), selon les méthodes décrites ci-dessous pour la préparation des composés (I') à partir des composés (I").

Les composés de formules (L6) peuvent être préparés à partir du dérivé 2-bromo benzothiazole (L5) par traitement avec un dérivé NH2R9, par exemple, dans un solvant tel le tétrahydrofurane, à une température voisine de 20°C. Le substituant R9 représente un radical alkyle ou cycloalkyle éventuellement substitué par un radical alcoxy, hétérocycloalkyle ou NR3R4 (R3 et R4 tels que définis ci-dessus).

Les composés de formules (L5) peuvent être préparés à partir du thiocyanate de 2-amino-1,3-benzothiazol-6-yle (K) (composé commercial), par exemple, par traitement avec un nitrite d'alkyle et du bromure cuivreux dans un solvant tel l'acétonitrile, à une température voisine de 0-20°C, selon la méthode décrite par Jagabandhu Das et. al. dans J. Med. Chem. 2006, 49, 6819-6832.

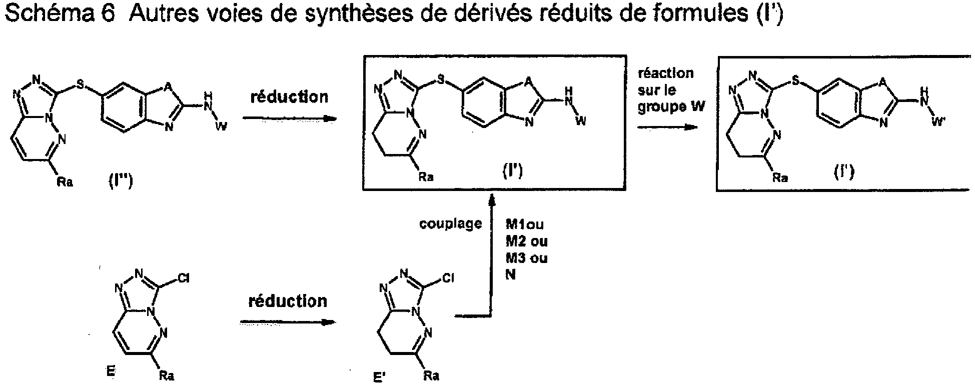

Selon le schéma 6 ci-dessus, les benzothiazoles de formule générale (I') peuvent également être préparés, à partir des composés de formule (I"), par réduction, par exemple, avec du borohydrure de sodium, dans un solvant tel l'éthanol, à une température voisine de 80°C ou bien par réduction avec du zinc (0) en présence d'acide acétique, à une température voisine de 20°C.

Alternativement les composés (I') peuvent également être préparés, à partir des composés de formule (E') par couplage avec les composés de type M1, M2, M3 ou N, obtenus en tant qu'intermédiaires par réduction des composés L1, L2, L3 ou K in situ, comme décrit ci-dessus au schéma 2. Les composés de type M1, M2 ou M3 peuvent aussi être isolés et utilisés pour le couplage avec (E'). Les composés (E') peuvent être obtenus à partir des composés de formule (E) par réduction, par exemple, par réduction avec du zinc (0) en présence d'acide acétique, à une température voisine de 20°C.

Alternativement les composé (I') peuvent également être préparés à partir d'autres composés (I') par transformation du groupement W en groupement W' de même nature que défini ci-dessus pour W et selon le type de réactions défini au schéma 2 : les transformations de 2d'/2d " en 2a'/2a" et en 2c'/2c", les transformation de 2a'/2a" en 2d'/2d" et en 2b'12b".

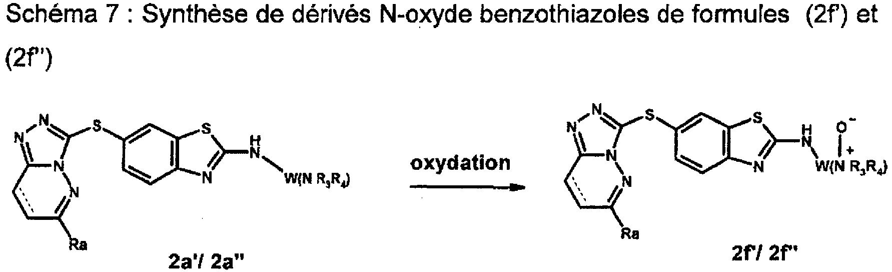

Selon le schéma 7 ci-dessus, les benzothiazoles de formule générale (2f') et (2f") peuvent être préparés respectivement à partir de composés de formule (2a') et (2a") dont le substituant W contient une fonction basique du type NR3R4 (R3 et R4 tels que définis ci-dessus avec R3 et R4 différents de H), par oxydation, par exemple, avec du périodate de sodium, en présence d'acide acétique, à une température voisine de 20°C.

Parmi les produits de départs de formule A, B, J, K, O, P, Q, R, S, U, V, W certains sont connus et peuvent être obtenus soit commercialement, soit selon les méthodes usuelles connues de l'homme du métier, par exemple à partir de produits commerciaux.

Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyles, trityle, benzyle, tert-butoxycarbonyle, BOC, benzyloxycarbonyle, phtalimido ou d'autres radicaux connus dans la chimie des peptides,

Les fonctions acide peuvent être protégées par exemple sous forme d'esters formés avec les esters facilement clivables tels que les esters benzyliques ou ter butyliques ou des esters connus dans la chimie des peptides.

On trouvera une liste de différents groupements protecteurs utilisables dans les manuels connus de l'homme du métier et par exemple dans le brevet BF 2 499 995.

On peut noter que l'on peut soumettre, si désiré et si nécessaire, des produits intermédiaires ou des produits de formule (I"b) ainsi obtenus par les procédés indiqués ci-dessus, pour obtenir d'autres intermédiaires ou d'autres produits de formule (I"b), à une ou plusieurs réactions de transformations connues de l'homme du métier telles que par exemple :
a) une réaction d'estérification de fonction acide,
b) une réaction de saponification de fonction ester en fonction acide,
c) une réaction de réduction de la fonction carboxy libre ou estérifié en fonction alcool,
d) une réaction de transformation de fonction alcoxy en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formule (I) ainsi obtenus étant sous toutes les formes isomères possibles racémiques, énantiomères et diastéréoisomères.

Les réactions a) à g) peuvent être réalisées dans les conditions usuelles connues de l'homme du métier telles que, par exemple, celles indiquées ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
   La réaction de saponification peut être réalisée selon les méthodes usuelles connues de l'homme du métier, telles que par exemple dans un solvant tel que le méthanol ou l'éthanol, le dioxane ou le diméthoxyéthane, en présence de soude ou de potasse.
c) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
d) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
e) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
f) Les produits décrits ci-dessus peuvent, si désiré, faire l'objet de réactions de salification par exemple par un acide minéral ou organique ou par une base minérale ou organique selon les méthodes usuelles connues de l'homme du métier : une telle réaction de salification peut être réalisée par exemple en présence d'acide chlorhydrique par exemple ou encore d'acide tartrique, citrique ou méthane sulfonique, dans un alcool tel que par exemple l'éthanol ou le méthanol
g) Les éventuelles formes optiquement actives des produits décrits ci-dessus peuvent être préparées par dédoublement des racémiques selon les méthodes usuelles connues de l'homme du métier.

Les produits de formule (I"b) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.

Les produits de la présente invention sont notamment utiles pour la thérapie de tumeurs.

Les produits de l'invention peuvent également ainsi augmenter les effets thérapeutiques d'agents anti-tumoraux couramment utilisés.

Ces propriétés justifient leur application en thérapeutique et l'invention a particulièrement pour objet à titre de médicaments, les produits de formule (I"b) telle que définie ci-dessus, lesdits produits de formule (I"b) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I"b).

L'invention concerne aussi des compositions pharmaceutiques contenant à titre de principe actif l'un au moins des produits de formule (I"b) tels que définis ci-dessus ou un sel pharmaceutiquement acceptable de ce produit et, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

La présente invention a également pour objet l'utilisation des produits de formule (I"b) tels que définis ci-dessus ou de sels pharmaceutiquement acceptables de ces produits pour la préparation d'un médicament destiné à l'inhibition de l'activité d'une protéine kinase.

La présente invention a également pour objet l'utilisation de produits de formule (I"b) tels que définis ci-dessus pour la préparation d'un médicament destiné au traitement ou à la prévention d'une maladie caractérisée par le dérèglement de l'activité d'une protéine kinase.

Un tel médicament peut notamment être destiné au traitement ou à la prévention d'une maladie chez un mammifère.

La présente invention a également pour objet l'utilisation définie ci-dessus dans laquelle la protéine kinase est une protéine tyrosine kinase.

La présente invention a également pour objet l'utilisation définie ci-dessus dans laquelle la protéine tyrosine kinase est MET ou ses formes mutantes.

La présente invention a également pour objet l'utilisation définie ci-dessus dans laquelle la protéine kinase est dans une culture cellulaire.

La présente invention a également pour objet l'utilisation définie ci-dessus dans laquelle la protéine kinase est dans un mammifère.

La présente invention a notamment pour objet l'utilisation d'un produit de formule (I"b) tel que défini ci-dessus pour la préparation d'un médicament destiné à la prévention ou au traitement de maladies liées à une prolifération non contrôlée.

La présente invention a particulièrement pour objet l'utilisation d'un produit de formule (I"b) tel que défini ci-dessus pour la préparation d'un médicament destiné au traitement ou à la prévention d'une maladie choisie dans le groupe suivant : troubles de la prolifération de vaisseaux sanguins, troubles fibrotiques, troubles de la prolifération de cellules 'mesangial', désordres métaboliques, allergies, asthmes, thromboses, maladies du système nerveux, rétinopathie, psoriasis, arthrite rhumatoïde, diabète, dégénérescence musculaire et cancers.

La présente invention a ainsi tout particulièrement pour objet l'utilisation d'un produit de formule (I"b) tel que défini ci-dessus pour la préparation d'un médicament destiné au traitement ou à la prévention de maladies en oncologie et notamment destiné au traitement de cancers.

Parmi ces cancers, on s'intéresse au traitement de tumeurs solides ou liquides, au traitement de cancers résistant à des agents cytotoxiques

Les produits de la présente invention cités peuvent notamment être utilisés pour le traitement de tumeurs primaires et/ou de métastases en particulier dans les cancers gastriques, hépatiques, rénaux, ovariens, du colon, de la prostate, du poumon (NSCLC et SCLC), les glioblastomes, les cancers de la thyroïde, de la vessie, du sein, dans les mélanomes, dans les tumeurs hématopoiétiques lymphoïdes ou myéloïdes, dans les sarcomes, dans les cancers du cerveau, du larynx, du système lymphatique, cancers des os et du pancréas.

La présente invention a aussi pour objet l'utilisation des produits de formule (I"b) telle que définie ci-dessus pour la préparation de médicaments destinés à la chimiothérapie de cancers.

De tels médicaments destinés à la chimiothérapie de cancers peuvent être utilisés seuls ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

Comme inhibiteurs de kinases, on peut citer la butyrolactone, le flavopiridol et la 2(2-hydroxyéthylamino)-6-benzylamino-9-méthylpurine appelée olomucine.

La présente invention a encore pour objet à titre de produits industriels nouveaux, les intermédiaires de synthèse de formules E', M1, M2 et M3 tels que définis ci-dessus et rappelés ci-après : 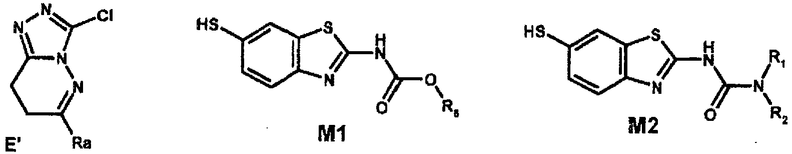 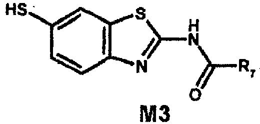 dans lesquels R6 représente un radical alkyle éventuellement substitué par un groupe NR3R4 ou OCH₃, un radical phényle, -(CH2)n-phényle, et n représente un entier de 1 à 2, ; R7 représente un radical cycloalkyle ou alkyle éventuellement substitué par un radical NR3R4 ou OCH₃ et Ra, R1, R2, R3 et R4 ont les significations indiquées ci-dessus.

Les exemples suivants qui sont des produits de formule (I"b) illustrent l'invention sans toutefois la limiteur.

### Partie expérimentale

La nomenclature des composés de cette présente invention a été effectuée avec le logiciel ACDLABS version 10.0.

Four micro-onde utilisé :
Biotage, Initiator EXP-EU, 300W max, 2450MHz

Les spectres de RMN 1 H à 400 MHz et 1 H à 300 MHz ont été effectués sur spectromètre BRUKER AVANCE DRX-400 ou BRUKER AVANCE DPX-300 avec les déplacements chimiques (δ en ppm) dans le solvant diméthylsulfoxide-d6(DMSO-d6) référencé à 2,5ppm à la température de 303K.

Les spectres de Masse ont été réalisés soit par analyse:
- LC-MS-DAD-ELSD (MS=Waters ZQ)
- LC-MS-DAD-ELSD (MS=Platform II Waters Micromass)
- UPLC-MS-DAD-ELSD (MS=Quattro Premier XE Waters)
DAD longueur d'onde considérée λ=210-400 nm
ELSD : Sedere SEDEX 85 ; nebulisation temperature=35°C ;nebulisation pressure=3,7 bars

### Exemple 1 :

### (6-{[6-(4-fluorophényl)[1,2,4]triazolo[4,3-b]pyridazin-3-yl]sulfanyl}-1,3-benzothiazol-2-yl)carbamate de méthyle

a) Le (6-{[6-{4-fluorophényl)[1,2,4]triazolo[4,3-b]pyridazin-3-yl]sulfanyl}-1,3-benzothiazol-2-yl)carbamate de méthyle peut être préparé de la manière suivante :
   à une suspension de 242mg de 3-chloro-6-(4-fluorophényle)-1,2,4-triazolo[4,3-b]pyridazine et de 100mg de (6-sulfanyl-1,3-benzothiazol-2-yl)carbamate de méthyle dans 3 cm³ de N,N-diméthylformamide à 20°C on ajoute 0,14cm³ de triéthylamine et 31mg de borohydrure de sodium. La suspension violette-brune partiellement soluble est agitée à 95°C pendant 2h. La solution est refroidie à 20°C puis reprise dans un mélange 50/50 d'eau et d'acétate d'éthyle. La suspension résultante est essorée pour donner 155mg d'un insoluble blanc-crème qui contient du produit attendu avec 70% de (6-sulfanyl-1,3-benzothiazol-2-yl)carbamate de méthyle. L'extraction des eaux-mères à l'acétate d'éthyle suivie du séchage des phases organiques sur sulfate de magnésium, filtration et concentration à sec au rotavapor donne une huile semi-cristallisée qui est ensuite filtrée. On obtient ainsi 48mg d'une poudre blanche contenant >80% de (6-{[6-(4-fluorophényl)[1,2,4]triazolo[4,3-b]pyridazin-3-yl]sulfanyl}-1,3-benzothiazol-2-yl)carbamate de méthyle.

   Les 155mg de produit contenant 70% de (6-sulfanyl-1,3-benzothiazol-2-yl)carbamate de méthyle sont remis en réaction avec 50mg de 3-chloro-6-(4-fluorophényle)-1,2,4-triazolo[4,3-b]pyridazine dans le N,N-diméthylformamide contenant 0,07 cm³ de triéthylamine et 15mg de borohydrure de sodium. La suspension violette-brune partiellement soluble est agitée à 95°C pendant 2h. La solution est refroidie à 20°C puis reprise dans un mélange 50/ 50 d'eau et d'acétate d'éthyle. La suspension résultante est filtrée pour donner 112mg d'un insoluble blanc-crème contenant le (6-{[6-(4-fluorophényl)[1,2,4]triazolo[4,3-b]pyridazin-3-yl]sulfanyl}-1,3-benzothiazol-2-yl)carbamate de méthyle.
   On rassemble les lots de 48mg et de 112mg et on purifie par chromatographie sur colonne Biotage Si 12M+ en éluant avec un gradient de 95/5 puis de 90/10 de dichlorométhane et d'une solution 38/17/2 de dichlorométhane/ méthanol/ ammoniaque aqueux.
   On obtient 56mg de (6-{[6-(4-fluorophényl)[1,2,4]triazolo[4,3-b]pyridazin-3-yl]sulfanyl)-1,3-benzothiazol-2-yl)carbamate de méthyle sous forme d'une poudre blanche dont les caractéristiques sont les suivantes :
   POINT DE FUSION > 270°C (Köfler)
   SPECTRE RMN 1 H (400 MHz, δ ppm) (DMSO-d6): 3,78 (s, 3H) ; 7,40 (t, J = 9,0 Hz, 2H) ; 7,54 (dd, J = 2,0 et 8,0 Hz, 1H) ; 7,65 (d, J = 8,0 Hz, 1H) ; 8,03 (d, J = 9,5 Hz, 1H) ; 8,10 (dd, J = 5,0 et 9,0 Hz, 2H) ; 8,21 (d, J =2,0 Hz, 1 H) ; 8,51 (d, J = 9,5 Hz, 1 H) ; 12,15 (m étalé, 1 H).
   SPECTRE DE MASSE: LC-MS-DAD-ELSD : 451(-)=(M-H)(-); 453(+)=(M+H)(+)
b) Le (6-sulfanyl-1,3-benzothiazol-2-yl)carbamate de méthyle peut être préparé de la manière suivante :
   à une suspension blanche de 280mg de (6-thiocyanato-1,3-benzothiazol-2-yl)carbamate de méthyle dans 11 cm³ d'éthanol à 20°C, on ajoute une solution de 5mg de dihydrogénophosphate de potassium dans 1,1 cm³ d'eau à 20°C, suivi de 480mg de DL-dithiothreitol. La suspension blanche est agitée 18h au reflux. On refroidit le mélange réactionnel à 20°C puis on additionne 10 cm³ d'eau et on agite pendant 15 minutes. Le précipité est essoré puis lavé à grands volumes d'eau. On obtient 231mg de (6-sulfanyl-1,3-benzothiazol-2-yl)carbamate de méthyle sous forme d'une poudre crème dont les caractéristiques sont les suivantes :
      SPECTRE DE MASSE: LC-MS-DAD-ELSD : 239(-)=(M-H)(-) ; 241(+)=(M+H)(+)
c) Le (6-thiocyanato-1,3-benzothiazol-2-yl)carbamate de méthyle peut être préparé de la manière suivante :
   à une solution verte de 1g de thiocyanate de 2-amino-1,3-benzothiazol-6-yle commercial dans 12 cm³ de pyridine à 0°C, on ajoute 0,467 cm³ de chlorocarbonate de méthyle à la seringue en maintenant la température à 0°C.
      La suspension est agitée 2h à 20°C avant ajout de 6 cm³ d'un mélange 50/50 d'eau et d'acétate d'éthyle. On filtre la poudre blanche obtenue sur fritté puis on la lave successivement à l'eau et à l'acétate d'éthyle.
      On obtient 816mg d'une poudre blanche de (6-thiocyanato-1,3-benzothiazol-2-yl)carbamate de méthyle sous forme d'une poudre blanche dont les caractéristiques sont les suivantes :
      SPECTRE DE MASSE: LC-MS-DAD-ELSD : 264(-)=(M-H)(-) ; 266(+)=(M+H)(+)
d) Le 3-chloro-6-(4-fluorophényl)-1,2,4-triazolo[4,3-b]pyridazine peut être préparé de la manière suivante :
   dans un tube pour microonde muni d'une agitation magnétique, on introduit à 20°C 700mg de 6-(4-fluorophényle)-1,2,4-triazolo[4,3-b]pyridazin-3-ol et 3,5 cm³ d' oxy chlorure de phosphore. Le mélange réactionnel est alors chauffé au microonde pendant 1 h à 150°C avant ajout de 100 cm³ d'une solution aqueuse saturée d'hydrogénocarbonate de sodium puis ajout supplémentaire d'hydrogénocarbonate de sodium pour neutraliser le milieu. Le mélange obtenu est extrait par 3x100 cm³ d'acétate d'éthyle. Les phases organiques sont rassemblées puis séchées sur sulfate de magnésium et concentrées à sec pour donner 696mg d'un solide orange que l'on chromatographie sur une cartouche Analogix 40g de silice 50µm (élution par du dichlorométhane pur puis par un mélange 80/20 de dichlorométhane/ acétate d'éthyle). On obtient ainsi 597mg de 3-chloro-6-(4-fluorophényle)-1,2,4-triazolo[4,3-b]pyridazine sous forme d'un solide beige dont les caractéristiques sont les suivantes :
      SPECTRE RMN 1H (400 MHz ; δ ppm) (DMSO-d6): 7,47 (t, J = 9,0 Hz, 2H) ; 8,07 (d, J = 9,5 Hz, 1H) ; 8,21 (dd, J = 5,0 et 9,0 Hz, 2H) ; 8,51 (d, J = 9,5 Hz, 1 H).
      SPECTRE DE MASSE: LC-MS-DAD-ELSD : 249(+)/...=(M+H)(+)/...(1 Cl present)
e) Le 6-(4-fluorophényl)-1,2,4-triazolo[4,3-b]pyridazin-3-ol peut être préparé de la manière suivante:
   à un mélange de 4,3g de 3-chloro-6-(4-fluorophényle)pyridazine dans 70 cm³ de butanol, à 20°C on ajoute 4,6g de chlorhydrate d'hydrazinecarboxamide et 5,7 cm³ de triéthylamine. Le mélange résultant est chauffé à 140°C pendant 65h puis refroidi à 20°C avant ajout de 300 cm³ de dichlorométhane. Le mélange réactionnel est ensuite lavé par 150 cm³ d'eau déminéralisée. Les phases organiques sont ensuite rassemblées puis séchées sur sulfate de magnésium et concentrées à sec pour donner 4,93g d'un solide orange. Ce solide est ensuite repris dans de l'oxyde de diéthyle puis essoré pour donner 2,5g de 6-(4-fluorophényl)-1,2,4-triazolo[4,3-b]pyridazin-3-ol sous forme d'un solide jaune dont les caractéristiques sont les suivantes :
      SPECTRE DE MASSE: LC-MS-DAD-ELSD : 231(+)/...=(M+H)(+)/...(1 Cl present)
f) Le 3-chloro-6-(4-fluorophényl)pyridazine peut être préparé de la manière suivante:
   à une solution de 300mg de 3-6-dichloropyrazine commerciale dans 12cm³ de dioxane sont ajoutés 140mg d'acide 4-fluorobenzène boronique et 231mg d'hydrogénocarbonate de sodium dans 7 cm³ d'eau déminéralisée. Le milieu est dégazé par barbotage d'argon pendant 5 minutes puis 115mg de tetrakis (triphenylphosphine) palladium (0) sont ajoutés. Le mélange obtenu est chauffé à 115°C pendant 1h30 puis refroidi à 20°C avant ajout de 20 cm³ d'eau déminéralisée. Le précipité formé est essoré puis lavé à l'eau déminéralisée.
      Après séchage on obtient 213mg d'un solide rose. L'extraction des phases aqueuses par 40 cm³ de dichlorométhane suivie du séchage sur sulfate de magnésium et concentration à sec de la phase organique donne 213mg d'une poudre beige.
      Les 2 solides sont rassemblés et chromatographiés sur une cartouche Analogix 12g de silice 50µm - (élution : dichlorométhane). On récupère ainsi 285mg de produit attendu que l'on chromatographie à nouveau dans les mêmes conditions pour donner 175mg de 3-chloro-6-(4-fluorophényle)pyridazine sous forme d'un solide blanc dont les caractéristiques sont les suivantes :
      SPECTRE DE MASSE : LC-MS-DAD-ELSD : 209(+)/...=(M+H)(+)/...(1 Cl present)

### Exemple 2 :

### (6-{[6-(4-fluorophényl)[1,2,4]triazolo[4,3-b]pyridazin-3-yl]sulfanyl}-1,3-benzothiazol-2-yl)carbamate de 1,1-diméthyléthyle

a) Le (6-{[6-(4-fluorophényl)[1,2,4]triazolo[4,3-b]pyridazin-3-yl]sulfanyl}-1,3-benzothiazol-2-yl)carbamate de 1,1-diméthyléthyle peut être préparé comme dans l'exemple 1a mais à partir de 56mg de (6-sulfanyl-1,3-benzothiazol-2-yl)carbamate de 1,1-diméthyléthyle et de 50mg de 3-chloro-6-(4-fluorophényl)-1,2,4-triazolo[4,3-b]pyridazine. On chromatographie sur Biotage Quad 12/25 (KP-SIL, 60A; 32-63µM) en éluant avec dichlorométhane/solution B 95/5 (solution B = dichlorométhane/ méthanol/ ammoniaque 38/17/2). On obtient 27 mg (6-{[6-(4-fluorophényl)[1,2,4]triazolo[4,3-b]pyridazin-3-yl]sulfanyl}-1,3-benzothiazol-2-yl)carbamate de 1,1-diméthyléthyle sous forme d'une poudre jaune dont les caractéristiques sont les suivantes :
   SPECTRE DE MASSE : LC-MS-DAD-ELSD : 495(+)=(M+H)(+)
   SPECTRE RMN : 1H NMR (400 MHz, DMSO-d6) δppm 1,50 (s, 9 H) 7,40 (t, J=9 Hz, 2 H) 7,54 (dd, J=8,3, 2,0 Hz, 1 H) 7,65 (d,J=8,3 Hz, 1 H) 8,02 (d, J=9,8 Hz, 1 H) 8,10 (dd, J=9,0, 5,6 Hz, 2 H) 8,19 (d, J=2,0 Hz, 1H) 8,51 (d, J=9,8 Hz, 1 H) 11,82 (br, s,, 1 H)
b) Le (6-sulfanyl-1,3-benzothiazol-2-yl)carbamate de 1,1-diméthyléthyle peut être préparé comme dans l'exemple 1b mais à partir de 615mg de (6-thiocyanato-benzothiazol-2-yl)carbamate de 1,1-diméthyléthyle, de 10mg de dihydrogénophosphate de potassium et de 926mg de DL-dithiothreitol. On obtient ainsi 659mg de (6-sulfanyl-1,3-benzothiazol-2-yl)carbamate de 1,1-diméthyléthyle sous forme d'une poudre blanche dont les caractéristiques sont les suivantes :
   SPECTRE DE MASSE : LC-MS-DAD-ELSD : 281(-)=(M-H)(-) ; 227(+)=(M+H)(-)- tBu
c) Le (6-thiocyanato-benzothiazol-2-yl)carbamate de 1,1-diméthyléthyle peut être préparé de la manière suivante :
   à un mélange de 1g de 6-thiocyanato-benzothiazol-2-ylamine commercial et de 2 cm³ de triéthylamine dans 20 cm³ de dichlorométhane à 0°C sous argon, on additionne 2,1g de dicarbonate di-tert butyle et on agite le mélange obtenu 1 heure à 0°C. On rajoute alors 147 mg de N,N-diméthylpyridin-4-amineet le mélange résultant est ensuite ramené progressivement à 20°C sur 2 heures, sous agitation. La solution limpide verte est coulée sur de l'eau et extraite à l'acétate d'éthyle. On obtient 1,765g d'une poudre jaune que l'on purifie par chromatographie sur Biotage Quad 12/25 (KP-SIL, 60A; 32-63µm) en éluant avec un gradient cyclohexane /acétate d'éthyle 95/5, 90/10, 85/15, 80/20, 70/30, 60/40. On obtient 1,058g de (6-thiocyanato-benzothiazol-2-yl) carbamate de 1,1-diméthyléthyle sous forme d'une poudre jaune dont les caractéristiques sont les suivantes :
      SPECTRE DE MASSE : LC-MS-DAD-ELSD : 308(+)=(M+H)(+)

### Exemple 3 :

### 6-{[6-(4-fluorophényl)[1,2,4]triazolo[4,3-b]pyridazin-3-yl]sulfanyl}-1,3-benzothiazol-2-amine

Le 6-{[6-(4-fluorophényl)[1,2,4]triazolo[4,3-b]pyridazin-3-yl]sulfanyl}-1,3-benzothiazol-2-amine peut être préparé de la manière suivante :
à un mélange de 127mg de (6-{[6-(4-fluorophényl)[1,2,4]triazolo[4,3-b]pyridazin-3-yl]sulfanyl}-1,3-benzothiazol-2-yl)carbamate de 1,1-diméthyléthyle dans 5 cm³ de dichlorométhane agité à 20°C, on rajoute 4x0,1 cm³ d'acide trifluoro-acétique (à 10% d'anisole) sur 7h, jusqu'à disparition du produit de départ. On concentre alors le mélange réactionnel sous pression réduite pour récupérer 186,5mg de poudre jaune que l'on purifie par chromatographie sur Biotage Quad 12/25 (KP-SIL, 60A; 32-63µm) en éluant avec un gradient de dichlorométhane puis dichlorométhane/méthanol : 99,5/0,5, 99/1, 98,5/1,5, 98/2, 97,5/2,5, 97/3. On obtient ainsi 36,6mg de 6-{[6-(4-fluorophényl)[1,2,4]triazolo[4,3-b]pyridazin-3-yl]sulfanyl}-1,3-benzothiazol-2-amine sous forme d'une poudre blanchâtre dont les caractéristiques sont les suivantes :
   POINT DE FUSION >260°C (Köfler)
   SPECTRE RMN 66292V 1 H NMR (400 MHz, DMSO-d6) δppm 7,31 (d, J=8,3 Hz, 1 H) 7,35 - 7,48 (m, 3 H) 7,66 (s, 2 H) 7,98 (d, J=2,0 Hz, 1 H) 8,01 (d, J=9,8 Hz, 1 H) 8,12 (dd, J=8,8, 5,4 Hz, 2 H) 8,49 (d, J=9,8 Hz, 1 H)
   SPECTRE DE MASSE : LC-MS-DAD-ELSD : 393(-)=(M-H)(-) 395(+)=(M+H)(+)

### Exemple 9 :

### 1-(6-{[6-(4-fluorophényl)[1,2,4]triazolo[4,3-b]pyridazin-3-yl]sulfanyl}-1,3-benzothiazol-2-yl)-3-(2-morpholin-4-yléthyl)urée

a) Le 1-(6-{[6-(4-fluorophényl)[1,2,4]triazolo[4,3-b]pyridazin-3-yl]sulfanyl}-1,3-benzothiazol-2-yl)-3-(2-morpholin-4-yléthyl)urée a été préparé selon la méthode décrite dans l'exemple 1a mais à partir de 240mg de 3-chloro-6-(4-fluorophényle)-1,2,4-triazolo[4,3-b]pyridazine, de 338mg de 1-(2-morpholin-4-yléthyl)-3-(6-sulfanyl-1,3-benzothiazol-2-yl)urée, de 0,14 cm³ de triéthylamine et de 38mg de borohydrure de sodium. Le brut obtenu est chromatographié sur une cartouche Merck (30g de silice 15-40µm) en éluant avec du dichlorométhane. On récupère ainsi 117 mg de 1-(6-{[6-(4-fluorophényl)[1,2,4]triazolo[4,3-b]pyridazin-3-yl]sulfanyl}-1,3-benzothiazol-2-yl)-3-(2-morpholin-4-yléthyl)urée, sous forme d'un solide beige dont les caractéristiques sont les suivantes :
   SPECTRE RMN 1H NMR (400 MHz, DMSO-d6) δppm : 2,34 - 2,46 (m, 6 H) 3,23 - 3,29 (m, 2 H) 3,59 (t, J=3,9 Hz, 4 H) 6,77 (br, s,, 1 H) 7,41 (t, J=8,8 Hz, 2 H) 7,52 (dd, J=8,3, 2,0 Hz, 1 H) 7,59 (d, J=8,3 Hz, 1 H) 8,03 (d, J=9,8 Hz, 1 H) 8,11 (dd, J=8,8, 5,4 Hz, 2 H) 8,17 (d, J=2,0 Hz, 1 H) 8,51 (d, J=9,8 Hz, 1 H) 10,90 (br, s,, 1 H)
   SPECTRE DE MASSE LC-MS-DAD-ELSD : 549(-)=(M-H)(-) ; 551(+)=(M+H)(+)
b) Le 1-(2-morpholin-4-yléthyl)-3-(6-sulfanyl-1,3-benzothiazol-2-yl)urée a été préparé selon la méthode décrite à l'exemple 1b mais à partir de 900mg de thiocyanate de 2-{[(2-morpholin-4-yléthyl)carbamoyl]amino}-1,3-benzothiazol-6-yle, de 11 mg de dihydrogénophosphate de potassium et de 1,1g DL-dithiothreitol. On obtient ainsi 633mg de 1-(2-morpholin-4-yléthyl)-3-(6-sulfanyl-1,3-benzothiazol-2-yl)urée, sous forme d'un solide blanc dont les caractéristiques sont les suivantes :
   SPECTRE DE MASSE: LC-MS-DAD-ELSD : 337(-)=(M-H)(-) ; 339(+)=(M+H)(+)
c) Le thiocyanate de 2-{[(2-morpholin-4-yléthyl)carbamoyl]amino}-1,3-benzothiazol-6-yle peut être préparé de la manière suivante :
   à une solution de 1g de (6-thiocyanato-1,3-benzothiazol-2-yl)carbamate de phényle ester dans 30 cm³ de tétrahydrofurane, on ajoute 0,44 cm³ de 2-morpholin-4-yléthanamine à 20°C. Après 24h, le mélange réactionnel est évaporé à sec et le résidu obtenu chromatographié sur une cartouche Merck 70g (dépôt solide; élution avec un gradient dichlorométhane puis dichlorométhane/méthanol 90/10). On récupère ainsi 902mg de thiocyanate de 2-{[(2-morpholin-4-yléthyl)carbamoyl]amino}-1,3-benzothiazol-6-yle, sous forme d'une mousse incolore dont les caractéristiques sont les suivantes :
      SPECTRE DE MASSE : UPLC-MS-DAD-ELSD : 364(+)=(M+H)(+)
d) Le (6-thiocyanato-1,3-benzothiazol-2-yl)carbamate de phényle a été préparé de la manière suivante :
   à une solution de 2,5g de thiocyanate de 2-amino-1,3-benzothiazol-6-yle commercial dans 94 cm³ de tétrahydrofurane, on ajoute, à 20°C, 7,5g de chlorocarbonate de phényle puis 4,05g d'hydrogénocarbonate de sodium et 9,4 cm³ d'eau. Le mélange résultant est ensuite agité à 20°C pendant 20h puis extrait par 2x150 cm³ d'acétate d'éthyle. Les phases organiques sont rassemblées puis lavées par 3X50 cm³ d'une solution aqueuse saturée d'hydrogénocarbonate de sodium. La phase organique obtenue est séchée sur sulfate de magnésium puis concentrée à sec sous pression réduite. On reprend le résidu dans 50 cm³ d'eau puis on essore et sèche sous vide à 20°C. On obtient ainsi 3,45g de (6-thiocyanato-1,3-benzothiazol-2-yl)carbamate de phényle, sous forme d'un solide jaune pâle dont les caractéristiques sont les suivantes :
      SPECTRE DE MASSE : LC-MS-DAD-ELSD : 326(-)=(M-H)(-) ; 328(+)=(M+H)(+)

### Exemple 10 :

### 1-(2-morpholin-4-yléthyl)-3-[6-([1,2,4]triazolo[4,3-b]pyridazin-3-ylsulfanyl)-1,3-benzothiazol-2-yl]urée

a) Le 1-(2-morpholin-4-yléthyl)-3-[6-([1,2,4]triazolo[4,3-b]pyridazin-3-ylsulfanyl)-1,3-benzothiazol-2-yl]urée peut être préparé de la manière suivante : à une solution de 462mg de 1-(2-morpholin-4-yléthyl)-3-(6-sulfanyl-1,3-benzothiazol-2-yl)urée dans 5,5 cm³ de tétrahydrofurane, on ajoute 277mg de n-tributyl phosphine. Ce mélange est agité 1h à 20°C sous barbotage d'argon avant ajout de 176mg de 3-chloro-1,2,4-triazolo[4,3-b]pyridazine, 166mg de tert-butylate de potassium, 12mg de tétraphényldiphosphoxane et 11 cm³ de toluène et agitation 30 mn à 20°C sous barbotage d'argon. On additionne alors au mélange résultant 10mg de tris(1,5-diphénylpenta-1,4-dién-3-one)dipalladium(0) et on porte au reflux pendant 17h. On concentre le solvant sous pression réduite puis on reprend le résidu dans de l'eau et de l'acide chlorhydrique HCl 0,1N. Le mélange obtenu est alors extrait avec un mélange d'acétate d'éthyle/méthanol 90/10. On obtient 632mg d'huile jaune que l'on chromatographie sur Biotage Quad 12/25 (KP-SIL, 60A; 32-63µM) en éluant avec un gradient dichlorométhane puis dichlorométhane/méthanol 99/1, 98/2, 97/3, 96/4, 95/6, 92/8, 90/10, 80/20. On obtient ainsi 75 mg de 1-(2-morpholin-4-yléthyl)-3-[6-([1,2,4]triazolo[4,3-b]pyridazin-3-ylsulfanyl)-1,3-benzothiazol-2-yl]urée, sous forme d'une poudre beige dont les caractéristiques sont les suivantes :
   POINT DE FUSION: 252°C (Köfler)
   SPECTRE RMN 1 H NMR (400 MHz, DMSO-d6) δppm : 2.36 - 2.45 (m, 6 H) 3.24 - 3.36 (m, 2 H) 3.55 - 3.63 (m, 1 H)6.78 (br. s., 0 H) 7.38 - 7.48 (m, 0 H) 7.57 (d, J=8.5 Hz, 0 H) 8.04 (d, J=1.5 Hz, 0 H) 8.43 (dd, J=9.5,1.5 Hz, 0 H) 8.70 (dd, J=4.5, 1.5 Hz, 0 H) 10.85 - 10.95 (m, 0 H)
   SPECTRE DE MASSE : UPLC-MS-DAD-ELSD : 457(+)=(M+H)(+)
b) Le 3-chloro-1,2,4-triazolo[4,3-b]pyridazine peut être préparé de la manière suivante :
   un mélange de 436mg de [1,2,4]triazolo[4,3-b]pyridazin-3-ol dans 6 cm³ de trichlorure phosphoric est agité au reflux pendant 3h30. Le mélange réactionnel est coulé sur une solution aqueuse de soude 4N et le mélange obtenu est extrait avec un mélange acétate d'éthyle/méthanol 90/10. La phase aqueuse toujours acide (pH1) est amenée à pH11 par addition de soude concentrée puis extraite à nouveau par un mélange acétate d'éthyle/méthanol 90/10. Les phases organiques sont rassemblées puis séchées sur sulfate de magnésium, filtrées puis évaporées sous vide. On obtient ainsi 808mg de gomme blanchâtre. On reprend 640 mg de cette gomme dans de l'acétate d'éthyle et on lave la solution résultante à l'eau. La phase organique est séchée sur sulfate de magnésium, filtrée puis évaporée sous vide pour donner 281mg de 3-chloro-1,2,4-triazolo[4,3-b]pyridazine, sous forme d'une poudre blanche dont les caractéristiques sont les suivantes :
      SPECTRE DE MASSE : UPLC-MS-DAD-ELSD : 155(+)/...=(M+H)(+)/...(1 Cl present)
c) Le [1,2,4]triazolo[4,3-b]pyridazin-3-ol peut être préparé de la manière suivante :
   un mélange de 1,71g de 6-chloro[1,2,4]triazolo[4,3-b]pyridazin-3-ol commercial, de 1,90g de formiate d'ammonium et de 2,13g de Pd/C à 5% dans 50 cm³ de méthanol est agité au reflux pendant 3h. Le mélange réactionnel est alors filtré pour éliminer le catalyseur et le filtrat obtenu est concentré sous pression réduite pour donner 2,74g de poudre verdâtre que l'on chromatographie sur Biotage Quad 12/25 (KP-SIL, 60A; 32-63mM) en éluant avec un gradient dichlorométhane/éluant B: 95/5, 90/10, 85/15, 80/20 (éluant B = dichlorométhane/ méthanol/ ammoniaque 38/17/2). On obtient ainsi 440mg de [1,2,4]triazolo[4,3-b]pyridazin-3-ol, sous forme d'une poudre blanchâtre dont les caractéristiques sont les suivantes :
      SPECTRE DE MASSE: LC-MS-DAD-ELSD : 135(-)=(M-H)(-) ; 137(+)=(M+H)(+)

### Exemple 11 :

### 1-{2-[(2R,6S)-2,6-diméthylmorpholin-4-yl]éthyl}-3-(6-{[6-(4-fluorophényl) [1,2,4]triazolo [4,3-b]pyridazin-3-yl]sulfanyl}-1,3-benzothiazol-2-yl)urée

a) Le 1-{2-[(2R,6S)-2,6-diméthylmorpholin-4-yl]éthyl}-3-(6-{[6-(4-fluorophényl) [1,2,4]triazolo [4,3-b]pyridazin-3-yl]sulfanyl}-1,3-benzothiazol-2-yl)urée a été préparé selon la méthode décrite à l'exemple 1a mais à partir de 186mg de 3-chloro-6-(4-fluorophényle)-1,2,4-triazolo[4,3-b]pyridazine, de 366mg de 1-{2-[(2R,6S)-2,6-diméthylmorpholin-4-yl]éthyl}-3-(6-sulfanyl-1,3-benzothiazol-2-yl)urée, de 0,14 cm³ de triéthylamine et de 38mg de borohydrure de sodium. On obtient ainsi 73mg de 1-{2-[(2R,6S)-2,6-diméthylmorpholin-4-yl]éthyl}-3-(6-{[6-(4-fluorophényl)[1,2,4]triazolo[4,3-b]pyridazin-3-yl]sulfanyl}-1,3-benzothiazol-2-yl)urée sous forme poudre beige dont les caractéristiques sont les suivantes :
   POINT DE FUSION : 150°C (Köfler)
   SPECTRE RMN 1H NMR (400 MHz, DMSO-d6) δppm : 1,04 (d, J=6,4 Hz, 6 H) 1,63 (t, J=10,5 Hz, 2 H) 2,39 (t, J=6,1 Hz, 2 H) 2,73 - 2,80 (m, 2 H) 3,24 - 3,29 (m, 2 H) 3,56 (br, s,, 2 H) 6,76 (br, s,, 1 H) 7,41 (t, J=9,0 Hz, 2 H) 7,51 (dd, J=8,5 2,0Hz 1H) 7,6 (d, J= 8,5Hz 1 H) 8,03 (d, J=9,8 Hz, 1 H) 8,11 (dd, J=8,8, 5,4 Hz, 2 H) 8,16 (d, J=1,5 Hz, 1 H) 8,51 (d, J=9,8 Hz, 1 H) 10,90 (br, s,, 1 H)
   SPECTRE DE MASSE : LC-MS-DAD-ELSD : 579(+)=(M+H)(+)
b) Le 1-{2-[(2R,6S)-2,6-diméthylmorpholin-4-yl]éthyl}-3-(6-sulfanyl-1,3-benzothiazol-2-yl)urée a été préparé selon la méthode décrite dans l'exemple 1b mais à partir de 640mg de thiocyanate de 2-[({2-[(2R,6S)-2,6-diméthylmorpholin-4-yl]éthyl}carbamoyl)amino]-1,3-benzothiazol-6-yle, de 7mg de dihydrogénophosphate de potassium et de 729mg de DL-dithiothreitol. On obtient ainsi 597mg de 1-{2-[(2R,6S)-2,6-diméthylmorpholin-4-yl]éthyl}-3-(6-sulfanyl-1,3-benzothiazol-2-yl)urée sous forme d'un solide blanc dont les caractéristiques sont les suivantes :
   SPECTRE DE MASSE: LC-MS-DAD-ELSD : 365(-)=(M-H)(-) 367(+)=(M+H)(+)
c) Le thiocyanate de 2-[({2-[(2R,6S)-2,6-diméthylmorpholin-4-yl]éthyl} carbamoyl) amino]-1,3-benzothiazol-6-yle a été préparé selon la méthode décrite dans l'exemple 9c mais à partir de 654mg de (6-thiocyanato-1,3-benzothiazol-2-yl)carbamate de phényle et de 0,35 cm³ de 2-[(2R,6S)-2,6-diméthylmorpholin-4-yl]éthanamine. On obtient ainsi 783mg de thiocyanate de 2-[({2-[(2R,6S)-2,6-diméthylmorpholin-4-yl]éthyl} carbamoyl) amino]-1,3-benzothiazol-6-yle sous forme d'un solide blanc dont les caractéristiques sont les suivantes :
   SPECTRE DE MASSE: LC-MS-DAD-ELSD : 392(+)=(M+H)(+)

### Exemple 12 :

### (6-{[6-(4-fluorophényl)[1,2,4]triazolo[4,3-b]pyridazin-3-yl]sulfanyl}-1,3-benzothiazol-2-yl)carbamate de 2-morpholin-4-yléthyle

a) (6-{[6-(4-fluorophényl)[1,2,4]triazolo[4,3-b]pyridazin-3-yl]sulfanyl}-1,3-benzothiazol-2-yl)carbamate de 2-morpholin-4-yléthyle peut être préparé selon la méthode décrite à l'exemple 1a mais à partir de 240mg de 3-chloro-6-(4-fluorophényle)-1,2,4-triazolo[4,3-b]pyridazine, de 339 mg de (6-sulfanyl-1,3-benzothiazol-2-yl)carbamate de 2-morpholin-4-yléthyle, de 0,14 cm³ de triéthylamine et de 38mg borohydrure de sodium. Le résidu est chromatographié sur une cartouche Analogix de 40g de silice 15-40µm en éluant avec un gradient dichlorométhane à dichlorométhane/méthanol 95/5. On récupère ainsi 160mg de (6-{[6-(4-fluorophényl)[1,2,4]triazolo[4,3-b]pyridazin-3-yl]sulfanyl}-1,3-benzothiazol-2-yl)carbamate de 2-morpholin-4-yléthyle sous forme d'un solide jaune dont les caractéristiques sont les suivantes :
   POINT DE FUSION: 202°C (Köfler)
   SPECTRE RMN 1 H NMR (400 MHz, DMSO-d6) δppm : 2,44 (t, J=4,5 Hz 4 H) 2,59 (t, J=5,5 Hz, 2 H) 3,55 (t, J=4,5 Hz 4 H) 4,29 (tl, J=5,5 Hz, 2 H) 7,40 (t, J=8,8 Hz, 2 H) 7,54 (dl, J=8,3 Hz, 1 H) 7,60 - 7,71 (m, 1 H) 8,03 (d, J=9,8 Hz, 1 H) 8,10 (dd, J=8,8, 5,4 Hz, 2 H) 8,20 (br, s,, 1 H) 8,52 (d, J=9,8 Hz, 1 H) 12,18 (br, s,, 1 H)
   SPECTRE DE MASSE : LC-MS-DAD-ELSD : 550(-)=(M-H)(-) ; 552(+)=(M+H)(+)
b) Le (6-sulfanyl-1,3-benzothiazol-2-yl)carbamate de 2-morpholin-4-yléthyle a été préparé selon la méthode décrite dans l'exemple 1 b mais à partir de 547mg de ((6-thiocyanato-1,3-benzothiazol-2-yl)carbamate de 2-morpholin-4-yléthyle, de 12mg de dihydrogénophosphate de potassium et de 1,16g de DL-dithiothreitol. On obtient ainsi 885mg de (6-sulfanyl-1,3-benzothiazol-2-yl)carbamate de 2-morpholin-4-yléthyle sous forme d'un solide blanc dont les caractéristiques sont les suivantes :
   SPECTRE DE MASSE: LC-MS-DAD-ELSD : 338(-)=(M-H)(-) ; 340(+)=(M+H)(+)
c) Le (6-thiocyanato-1,3-benzothiazol-2-yl)carbamate de 2-morpholin-4-yléthyle a été préparé selon la méthode décrite à l'exemple 9c mais à partir de 654mg (6-thiocyanato-1,3-benzothiazol-2-yl)carbamate de phényle et de 0,27 cm³ de 2-morpholin-4-yléthanamine. Le résidu obtenu est chromatographié sur une cartouche Analogix de 40g de silice 15-40µm en éluant avec un gradient dichlorométhane puis dichlorométhane/ méthanol 95/5. On obtient ainsi 729mg de (6-thiocyanato-1,3-benzothiazol-2-yl)carbamate de 2-morpholin-4-yléthyle sous forme d'un solide blanc dont les caractéristiques sont les suivantes :
   SPECTRE DE MASSE: LC-MS-DAD-ELSD : 365(+)=(M+H)(+)

### Exemple 14 :

### 1-(6-{[6-(4-fluorophényl)[1,2,4]triazolo[4,3-b]pyridazin-3-yl]sulfanyl}-1,3-benzothiazol-2-yl)-3-[2-(4-méthylpipérazin-1-yl)éthyl]urée

Le 1-(6-{[6-(4-fluorophényl)[1,2,4]triazolo[4,3-b]pyridazin-3-yl]sulfanyl}-1,3-benzothiazol-2-yl)-3-[2-(4-méthylpipérazin-1-yl)éthyl]urée a été obtenu à l'exemple 13 et a les caractéristiques sont les suivantes :
POINT DE FUSION : 176°C (Köfler)
SPECTRE RMN 1H NMR (400 MHz, DMSO-d6) δppm : 2,15 (s, 3 H) 2,23 - 2,46 (m, 10 H) 3,20 - 3,28 (m, 2 H) 6,74 (br, s,, 1 H) 7,40 (t, J=9,0 Hz, 2 H) 7,52 (dd, J= 8,7, 2 Hz1 H) 7,59 (d, J=8,7 Hz 1 H) 8,02 (d, J=9,8 Hz, 1 H) 8,11 (dd, J=8,8, 5,4 Hz, 2 H) 8,16 (d, J=2,0 Hz, 1 H) 8,51 (d, J=9,8 Hz, 1 H) 10,91 (br, s,, 1 H)
SPECTRE DE MASSE : LC-MS-DAD-ELSD : 562(-)=(M-H)(-) ; 564(+)=(M+H)(+)

### Exemple 15 :

### N-(6-{[6-(4-fluorophényl)[1,2,4]triazolo[4,3-b]pyridazin-3-yl]sulfanyl}-1,3-benzothiazol-2-yl)-4-morpholin-4-ylbutanamide

a) Le N-(6-{[6-(4-fluorophényl)[1,2,4]triazolo[4,3-b]pyridazin-3-yl]sulfanyl}-1,3-benzothiazol-2-yl)-4-morpholin-4-ylbutanamide peut être préparé selon la méthode décrite dans l'exemple 1a mais à partir de 202mg de 4-morpholin-4-yl-N-(6-sulfanyl-1,3-benzothiazol-2-yl)butanamide, de 150mg de 3-chloro-6-(4-fluorophényle)-1,2,4-triazolo[4,3-b]pyridazine, de 0,125 cm³ de triéthylamine et de 14mg borohydrure de sodium. Le brut obtenu est chromatographié sur une cartouche Merck (25g de silice 15-40µm) en éluant avec du dichlorométhane puis un mélange dichlorométhane/ méthanol/ ammoniaque 38/17/3. On récupère ainsi 71 mg de N-(6-{[6-(4-fluorophényl) [1,2,4]triazolo[4,3-b]pyridazin-3-yl]sulfanyl}-1,3-benzothiazol-2-yl)-4-morpholin-4-ylbutanamide, sous forme d'une poudre beige dont les caractéristiques sont les suivantes :
   POINT DE FUSION : 220°C (Köfler)
   SPECTRE DE MASSE : LC-MS-DAD-ELSD : 548(-)=(M-H)(-) ; 550(+)=(M+H)(+)
   SPECTRE RMN 1H NMR (400 MHz, DMSO-d6) δppm : 1,78 (quin, J=7,1 Hz, 2 H) 2,23 - 2,36 (m, 6 H) 2,5 (2H) 3,47 (t, J=4,6 Hz, 3 H) 7,40 (t, J=8,8 Hz, 2 H) 7,57 (dd, J=8,5, 2,0 Hz, 1 H) 7,71 (d, J=8,5 Hz, 1 H) 8,03 (d, J=9,8 Hz, 1 H) 8,10 (dd, J=8,8, 5,4 Hz, 2 H) 8,24 (d, J=2 Hz, 1 H) 8,52 (d, J=9,8 Hz, 1 H) 12,41 (br, s,, 1 H)
b) Le 4-morpholin-4-yl-N-(6-sulfanyl-1,3-benzothiazol-2-yl)butanamide a été préparé selon la méthode décrite dans l'exemple 1b mais à partir de 906mg de 4-morpholin-4-yl-N-(6-thiocyanato-1,3-benzothiazol-2-yl]butanamide, de 14mg de dihydrogénophosphate de potassium et de 1,12g de DL-dithiothreitol. On obtient ainsi 71mg de 4-morpholin-4-yl-N-(6-sulfanyl-1,3-benzothiazol-2-yl)butanamide sous forme d'un solide blanc dont les caractéristiques sont les suivantes :
   SPECTRE DE MASSE : LC-MS-DAD-ELSD : 336(-)=(M-H)(-) ; 338(+)=(M+H)(+)
c) Le 4-morpholin-4-yl-N-(6-thiocyanato-1,3-benzothiazol-2-yl]butanamide peut être préparé de la manière suivante :
   à un mélange de 3,77g d'acide 4-morpholin-4-ylbutanoïque, de 7,5 cm³ de triéthylamine et de 6,90g de chlorhydrate de N-[3-(diméthylamino)propyl]-N'-éthylcarbodiimide dans 72 cm³ de dichlorométhane, on ajoute 4,86g de 1-hydroxybenzotriazole et 1,72g de N,N-diméthylpyridin-4-amine. Après 5min d'agitation à 20°C, on ajoute au mélange obtenu 3,73g de thiocyanate de 2-amino-1,3-benzothiazol-6-yle commercial puis on chauffe à reflux 23h avant retour à 20°C et concentration à sec du mélange réactionnel. Le résidu obtenu est chromatographié sur une cartouche Analogix de 400g en éluant avec du dichlorométhane puis un mélange dichlorométhane/méthanol 95/5. On récupère un mélange qui est purifié à nouveau sur une cartouche Merck de 90g dans le mêmes conditions d'élution. On récupère ainsi 1,76g de 4-morpholin-4-yl-N-(6-thiocyanato-1,3-benzothiazol-2-yl]butanamide sous forme d'un solide amorphe jaune dont les caractéristiques sont les suivantes :
      SPECTRE DE MASSE : LC-MS-DAD-ELSD : 363(+)=(M+H)(+)

### Exemple 17:

### 1-[2-(diéthylamino)éthyl]-3-(6-{[6-(4-fluorophényl)[1,2,4]triazolo[4,3-b]pyridazin-3-yl]sulfanyl}-1,3-benzothiazol-2-yl)urée

a) Le 1-[2-(diéthylamino)éthyl]-3-(6-{[6-(4-fluorophényl)[1,2,4]triazolo[4,3-b]pyridazin-3-yl]sulfanyl}-1,3-benzothiazol-2-yl)urée peut être préparé de la manière suivante :
   à une solution de 300mg de thiocyanate de 2-({[2-(diéthylamino)éthyl]carbamoyl} amino)-1,3-benzothiazol-6-yle dans 6cm³ d'éthanol, on fait barboter un courant d'argon pendant 5 minutes. On ajoute ensuite 6mg de dihydrogénophosphate de potassium dans 0,6cm³ d'eau, 396mg de DL-dithiothréitol et 204mg de 3-chloro-6-(4-fluorophényle)-1,2,4-triazolo[4,3-b]pyridazine. La réaction est chauffée au reflux pendant 19h, puis la solution est évaporée à sec sous vide. Le résidu est purifié sur une cartouche de silice Merck de 25 g par dépôt solide en éluant avec un gradient de 100% dichlorométhane à dichlorométhane/(dichlorométhane 38 / méthanol 17 / ammoniaque 2) 8/2. On obtient ainsi 225mg de 1-[2-(diéthylamino)éthyl]-3-(6-{[6-(4-fluorophényl)[1,2,4]triazolo[4,3-b]pyridazin-3-yl]sulfanyl}-1,3-benzothiazol-2-yl)urée sous forme d'une poudre jaune dont les caractéristiques sont les suivantes :
      POINT DE FUSION : 176°C (Köfler)
      SPECTRE DE MASSE : SPECTRE DE MASSE : LC/MS Electrospray sur WATERS UPLC - SQD : MH+ = 537 + ; MH- = 535 -
      SPECTRE RMN 1 H (400 MHz, DMSO-*d*6) δ ppm : 0.97 (t, *J*=7.3 Hz, 6 H) 2.50 (m partiellement masqué, 6 H) 3.20 (q, *J*=6.0 Hz, 2 H) 6.73 (m large, 1 H) 7.40 (t, *J*=8.8 Hz, 2 H) 7.52 (dd, , *J*=8.5, 2.4 Hz, 1 H) 7.59 (d, *J*=8.5 Hz, 1 H) 8.02 (d, *J*=9.8 Hz, 1 H) 8.11 (dd, *J*=8.8, 5.4 Hz, 2 H) 8.16 (d, *J*=2.4 Hz, 1 H) 8.51 (d, *J*=9.8 Hz, 1 H) 10.91 (m étalé, 1 H)
b) Le thiocyanate de 2-({[2-(diéthylamino)éthyl]carbamoyl}amino)-1,3-benzothiazol-6-yle peut être préparé selon la méthode décrite à l'exemple 9c mais à partir de 982mg de (6-thiacyanato-1,3-benzothiazol-2-yl)carbamate de phényle ester, 30cm³ de THF et 0,465 cm³ de N,N-diéthyléthylénediamine. Après purification sur une cartouche Merck de 30g de silice en éluant avec un gradient de 100% dichlorométhane à dichlorométhane/(dichlorométhane 38 / méthanol 17 / ammonique 2) 8/2, on obtient 896mg de thiocyanate de 2-({[2-(diéthylamino)éthyl]carbamoyl}amino)-1,3-benzothiazol-6-yle sous forme de solide blanc dont les caractéristiques sont les suivantes :
   SPECTRE DE MASSE : UPLC-MS-DAD-ELSD : 348=MH- ; 350=MH+
c) Le 3-chloro-6-(4-fluorophényle)-1,2,4-triazolo[4,3-b]pyridazine peut être préparé de la manière suivante :
   un mélange de 4,16g d'acide 4-fluorophényle boronique, 9,37g d'hydroxyde de barium octahydrate, 2,20g de [1,1'-bis (diphénylphosphino)ferrocène]di-chloropalladium (II) en complexe avec du dichlorométhane (1:1) et 5,1g de 3,6-dichloro[1,2,4]triazolo[4,3-b]pyridazine commerciale dans 40cm³ de N, N-diméthylformamide contenant 10cm³ d'eau est chauffé dans un bain à 80°C pendant 1,5h. La suspension marron-beige obtenue est refroidie à 20°C puis versée sur environ 200cm³ d'eau. L'insoluble est essoré et lavé successivement à l'eau et, à l'éther puis séché sous vide à 20°C. Le solide beige résultant est empâté dans du dichlorométhane, essoré et séché sous vide à 20°C. On obtient ainsi 1,24g de 3-chloro-6-(4-fluorophényle)-1,2,4-triazolo[4,3-b]pyridazine. Aux liqueurs mères combinées sont ajouté 30g de silice et le mélange est évaporé à sec sous vide. Ce résidu est déposé sur un lit de 10g de silice dans un filtre à verre frité et on élue au dichlorométhane. On récupère ainsi 1,60g additionnels de 3-chloro-6-(4-fluorophényle)-1,2,4-triazolo[4,3-b]pyridazine.

### Exemple 26 :

### 6-{[6-(4-fluorophényl)[1,2,4]triazolo[4,3-b]pyridazin-3-yl]sulfanyl}-N-(2-méthoxyéthyl)-1,3-benzothiazol-2-amine

a) Le 6-{[6-(4-fluorophényl)[1,2,4]triazolo[4,3-b]pyridazin-3-yl]sulfanyl}-N-(2-méthoxyéthyl)-1,3-benzothiazol-2-amine peut être préparé de la manière suivante :
   à un mélange de 263mg de (6-{[6-(4-fluorophényl)[1,2,4]triazolo[4,3-b]pyridazin-3-yl]sulfanyl}-1,3-benzothiazol-2-yl)carbamate de 1,1-diméthyléthyle et de 0,10cm³ de chloro-2-éthyle méthyle éther dans 3cm³ de N, N-diméthylformamide à 20°C, sont ajoutés 45mg d'hydrure de sodium à 60% dans l'huile. La suspension est chauffée dans un bain à 90°C un nuit. Le mélange réactionnel refroidi est co-évaporé plusieurs fois à sec avec du toluène. Le résidu obtenu est repris dans du méthanol. Après 15min d'empattage, la suspension jaune-brune est essorée. L'insoluble est lavé plusieurs fois avec du méthanol. Ce solide est traité avec 0,4cm³ d'acide trifluoroacétique dans 3 cm³ de dichlorométhane à 20°C pendant 2h. Le mélange réactionnel résultant est concentré à sec sous vide pour donner 116mg de 6-{[6-(4-fluorophényl)[1,2,4]triazolo[4,3-b]pyridazin-3-yl]sulfanyl}-N-(2-méthoxyéthyl)-1,3-benzothiazol-2-amine sous forme de poudre jaune beige dont les caractéristiques sont les suivantes
      POINT DE FUSION: 212°C (Köfler)
      SPECTRE DE MASSE : LC/MS Electrospray sur WATERS UPLC - SQD : MH+ = 453 + ; MH- = 451 -
      SPECTRE RMN 1H (400 MHz, DMSO-*d*6) δ ppm: Pour ce lot, tous les signaux sont larges avec :3.27 (s, 3 H) 3.51 (m, 4 H) 7.27 - 7.48 (m, 4 H) 7.95 - 8.03 (m, 2 H) 8.11 (m, 2 H) 8.28 (m, 1 H) 8.48 (d, *J*=9.8 Hz, 1 H)

### Exemple 30 :

### 6-{[6-(4-fluorophényl)[1,2,4]triazolo[4,3-b]pyridazin-3-yl]sulfanyl}-N-[2-(morpholin-4-yl)éthyl]-1,3-benzothiazol-2-amine

a) Le 6-{[6-(4-fluorophényl)[1,2,4]triazolo[4,3-b]pyridazin-3-yl]sulfanyl}-N-[2-(morpholin-4-yl)éthyl]-1,3-benzothiazol-2-amine peut être préparé de la manière suivante :
   à une solution de 636mg de [2-(morpholin-4-yl)éthyl](6-thiocyanato-1,3-benzothiazol-2-yl)carbamate de 1,1-diméthyléthyle dans 10cm³ d'éthanol dégazé à l'argon pendant 5min sont ajouté 8mg de dihydrogénophosphate dans 1 cm³ d'eau, 680mg de DL-dithiothreitol et 336 mg de 3-chloro-6-(4-fluorophényl)[1,2,4]triazolo[4,3-b]pyridazine, à 20°C. La suspension est agitée 18h au reflux. La réaction est placé au réfrigérateur une nuit puis le solide blanc-gris est essoré. Ce solide est lavé à l'éther diéthylique puis empâté dans le dichlorométhane et essoré. On obtient ainsi 222mg de 6-{[6-(4-fluorophényl)[1,2,4]triazolo[4,3-b]pyridazin-3-yl]sulfanyl}-N-[2-(morpholin-4-yl)éthyl]-1,3-benzothiazol-2-amine sous forme d'une poudre blanche grise dont les caractéristiques sont les suivantes :
      POINT DE FUSION : 280°C (Köfler)
      SPECTRE DE MASSE : LC/MS Electrospray sur WATERS UPLC - SQD : MH+ = 508 + ; MH- = 506 -
      SPECTRE RMN 1 H (400 MHz, DMSO-d6) δ ppm: Pour ce lot, tous les signaux sont larges avec : 2.40 (m, 4 H) 2.50 (m masqué, 2 H) 3.48 (m, 2 H) 3.55 (m, 4 H) 7.28 - 7.52 (m, 4 H) 7.96 - 8.03 (m, 2 H) 8.06 - 8.22 (m,3 H) 8.49 (d, *J=*10.0 Hz, 1 H)
b) Le [2-(morpholin-4-yl)éthyl](6-thiocyanato-1,3-benzothiazol-2-yl)carbamate de 1,1-diméthyléthyle peut être préparé de la manière suivante :
   à un mélange de 500mg de (6-thiocyanato-1,3-benzothiazol-2-yl)carbamate de 1,1-diméthyléthyle dans 7cm³ de N,N-diméthylformamide à 20°C, sont ajoutés 195mg d'hydrure de sodium à 60% dans l'huile. Après 5min, on ajoute 606mg de chlorhydrate de 4-(2-chloroéthyle)morpholine. Le milieu réactionnel est agité un week-end puis concentré à sec sous vide. Le résidu est purifié sur colonne Biotage Si-25 M par dépôt à sec en éluant avec un gradient dichlorométhane/solution B de 95/5 à 90/10 [Solution B : dichlorométhane/méthanol/ammoniaque (38/17/2)]. On obtient 647mg de [2-(morpholin-4-yl)éthyl](6-thiocyanato-1,3-benzothiazol-2-yl)carbamate de 1,1-diméthyléthyle engagé tel quel dans la suite des synthèses.
      SPECTRE DE MASSE : UPLC-MS-DAD-ELSD : 421=MH+ ; 321=(MH+)-TBoc+H

### Exemple 33 :

### (6-{[6-(4-fluorophényl)[1,2,4]triazolo[4,3-b]pyridazin-3-yl]sulfanyl}-1,3-benzothiazol-2-yl)carbamate de phényle

a) Le (6-{[6-(4-fluorophényl)[1,2,4]triazolo[4,3-b]pyridazin-3-yl]sulfanyl}-1,3-benzothiazol-2-yl)carbamate de phényle peut être préparé selon la méthode décrite à l'exemple 9d mais à partir de 630mg de 6-{[6-(4-fluorophényl)[1,2,4]triazolo[4,3-b]pyridazin-3-yl]sulfanyl}-1,3-benzothiazol-2-amine et de 0,9cm³ de chloroformiate de phényle dans 6cm³ de pyridine après 4h de contact à 20°C. On obtient ainsi 823mg de (6-{[6-(4-fluorophényl)[1,2,4]triazolo[4,3-b]pyridazin-3-yl]sulfanyl}-1,3-benzothiazol-2-yl)carbamate de phényle sous forme d'un solide beige dont les caractéristiques sont les suivantes :
   POINT DE FUSION > 265°C (Köfler)
   SPECTRE DE MASSE : LC/MS Electrospray sur WATERS UPLC - SQD : MH+ =515+
   SPECTRE RMN 1H (400 MHz, DMSO-*d*6) δ ppm : 7.24 - 7.33 (m, 3 H) 7.36 - 7.48 (m, 4 H) 7.56 (dd, *J*=8.5, 2.0 Hz, 1 H) 7.70 (d large, *J*=8.5 Hz, 1 H) 8.03 (d, *J*=9.8 Hz, 1 H) 8.10 (dd, *J*=9.0, 5.4 Hz, 2 H) 8.20 (s large, 1 H) 8.52 (d, *J*=9.8 Hz, 1 H) 12.68 (m étalé, 1 H)

Le 6-{[6-(4-fluorophényl)[1,2,4]triazolo[4,3-b]pyridazin-3-yl]sulfanyl}-1,3-benzothiazol-2-amine peut aussi être préparé selon la méthode décrite à l'exemple 17a mais à partir de 829mg 2-amino-6-thiocyanatobenzothiazole commercial, de 40cm³ d'éthanol, 20mg de dihydrogénophosphate de potassium dans 1cm³ d'eau, de 1,85g de DL-dithiothréitol et de 995mg de 3-chloro-6-(4-fluorophényl)[1,2,4]triazolo[4,3-b]pyridazine. On obtient ainsi 1,58g de 6-{[6-(4-fluorophényl)[1,2,4]triazolo[4,3-b]pyridazin-3-yl]sulfanyl}-1,3-benzothiazol-2-amine sous forme d'une solide marron claire dont les caractéristiques sont les suivantes :
POINT DE FUSION > 265°C (Köfler)

### Exemple 41 :

### N-(6-{[6-(4-fluorophényl)[1,2,4]triazolo[4,3-b]pyridazin-3-yl]sulfanyl}-1,3-benzothiazol-2-yl)acétamide

Le N-(6-{[6-(4-fluorophényl)[1,2,4]triazolo[4,3-b]pyridazin-3-yl]sulfanyl}-1,3-benzothiazol-2-yl)acétamide peut être préparé selon la méthode décrite à l'exemple 18b mais à partir de 273mg de 6-{[6-(4-fluorophényl)[1,2,4]triazolo[4,3-b]pyridazin-3-yl]sulfanyl}-1,3-benzothiazol-2-amine dans 2cm³ de pyridine et de 1cm³ d'anhydride acétique à 60°C pendant 4h. On obtient ainsi 207mg de N-(6-{[6-(4-fluorophényl)[1,2,4]triazolo[4,3-b]pyridazin-3-yl]sulfanyl}-1,3-benzothiazol-2-yl)acétamide sous forme d'un solide beige dont les caractéristiques sont les suivantes :
POINT DE FUSION > 255°C (Büchi B-545)
SPECTRE DE MASSE : LC/MS Electrospray sur WATERS UPLC - SQD :MH+ = 437 + ; MH- = 435 -
SPECTRE RMN 1H (400 MHz, DMSO-*d*6) δ ppm : 2.19 (s, 3 H) 7.40 (t, *J*=9.0 Hz, 2 H) 7.57 (dd, *J*=8.5, 2.1 Hz, 1 H) 7.72 (d, *J*=8.5 Hz, 1 H) 8.03 (d, *J*=9.8 Hz, 1 H) 8.06 - 8.15 (dd, *J*=9.0, 5.5 Hz, 2 H) 8.23 (d, *J*=2.1 Hz, 1 H) 8.52 (d, *J*=9.8 Hz, 1 H) 12.39 (s large, 1 H)

### Exemple 42 :

### 1-(6-{[6-(4-fluorophényl)[1,2,4]triazolo[4,3-b]pyridazin-3-yl]sulfanyl}-1,3-benzothiazol-2-yl)-3-[2-(4-oxidomorpholin-4-yl)éthyl]urée

a) Le 1-(6-{[6-(4-fluorophényl)[1,2,4]triazolo[4,3-b]pyridazin-3-yl]sulfanyl}-1,3-benzothiazol-2-yl)-3-[2-(4-oxidomorpholin-4-yl)éthyl]urée peut être préparé de la manière suivante :
   à un mélange de 200mg de 1-(6-{[6-(4-fluorophényl)[1,2,4]triazolo[4,3-b]pyridazin-3-yl]sulfanyl}-1,3-benzothiazol-2-yl)-3-[2-(morpholin-4-yl)éthyl]urée dans 5cm³ d'acide acétique à 20°C, sont ajoutés 116mg de périodate de sodium dans 0,5cm³ d'eau. Après une nuit, on filtre le précipité. La poudre blanche obtenue est un mélange contenant du produit oxydé et du produit de départ. Ce mélange est repris dans 10cm³ d'acide acétique avec 155mg de périodate de sodium dissous dans 1 cm³ d'eau. On laisse le mélange sous agitation une nuit, à 20°C. Le précipité est alors filtré puis successivement lavé à l'éther diisopropylique et à l'éther diéthylique. On obtient 155 mg de poudre blanche de 1-(6-{[6-(4-fluorophényl)[1,2,4]triazolo[4,3-b]pyridazin-3-yl]sulfanyl}-1,3-benzothiazol-2-yl)-3-[2-(4-oxidomorpholin-4-yl)éthyl]urée dont les caractéristiques sont les suivantes :
      POINT DE FUSION : 190°C (Köfler)
      SPECTRE DE MASSE : LC/MS Electrospray sur WATERS UPLC - SQD : MH+ = 567 + ; MH- = 565 -
      SPECTRE RMN 1 H (400 MHz, DMSO-*d*6) δ ppm : 3.49 - 4.07 (m, 12 H) 7.41 (t, *J*=8.8 Hz, 2 H) 7.53 (d, *J*=8.5, 2.0 Hz, 1 H) 7.55 (m masqué, 1 H) 7.61 (d, *J*=8.5 Hz, 1 H) 8.03 (d, *J*=9.8 Hz, 1 H) 8.12 (dd, *J*=8.8, 5.4 Hz, 2 H) 8.16 (d, *J*=2.0 Hz, 1 H) 8.51 (d, *J*=9.8 Hz, 1 H) 11.29 (m étalé, 1 H)

### Exemple 53:

### 6-{[6-(3-fluorophényl)[1,2,4]triazolo[4,3-b]pyridazin-3-yl]sulfanyl}-1,3-benzothiazol-2-amine

a) Le 6-{[6-(3-fluorophényl)[1,2,4]triazolo[4,3-b]pyridazin-3-yl]sulfanyl}-1,3-benzothiazol-2-amine peut être préparé selon la méthode décrite à l'exemple 17a mais à partir de 416mg de thiocyanate de 2-amino-1,3-benzothiazol-6-yle (commercial), de 23cm³ d'éthanol dégazé, de 9,3mg de dihydrogénophosphate de potassium dans 0,22cm³ d'eau, de 930mg de DL-dithiothréitol et de 413mg de 3-chloro-6-(3-fluorophényl)[1,2,4]triazolo[4,3-b]pyridazine. On obtient ainsi 242mg de 6-{[6-(3-fluorophényl)[1,2,4]triazolo[4,3-b]pyridazin-3-yl]sulfanyl}-1,3-benzothiazol-2-amine sous forme d'une poudre blanche dont les caractéristiques sont les suivantes :
   POINT DE FUSION : >255°C (Büchi B-545)
   SPECTRE DE MASSE: LC/MS Electrospray sur WATERS UPLC - SQD: MH+ = 395+ ; MH- = 393-
   SPECTRE RMN 1 H (300 MHz, DMSO-d6) δ ppm : 7.31 (d, *J*=8.5 Hz, 1 H) 7.43 (dd, *J*=8.5, 2.0 Hz, 1 H) 7.46 (m partiellement masqué, 1 H) 7.57 - 7.67 (m, 3 H) 7.84 (ddd, *J*=10.5, 2.6, 1.7 Hz, 1 H) 7.91 (d large, *J*=8.1 Hz, 1 H) 7.98 (d, *J*=2.0 Hz, 1 H) 8.04 (d, *J*=9.8 Hz, 1 H) 8.52 (d, *J*=9.8 Hz, 1 H)
b) Le 3-chloro-6-(3-fluorophényl)[1,2,4]triazolo[4,3-b]pyridazine peut être préparé selon la méthode décrite à l'exemple 17c mais à partir de 820mg d'acide 3-fluorophényle boronique, de 1,84g d'hydroxyde de barium octahydrate, de 0,43g de [1,1'-bis (diphénylphosphino)ferrocène]di-chloropalladium (II) en complexe avec du dichlorométhane (1:1) et 1g de 3,6-dichloro[1,2,4]triazolo[4,3-b]pyridazine commerciale dans 8cm³ de N, N-diméthylformamide dégazé et 1,96cm³ d'eau, après 1,5h à 80°C. On obtient ainsi 506mg de 3-chloro-6-(3-fluorophényl)[1,2,4]triazolo[4,3-b]pyridazine sous forme de poudre beige dont les caractéristiques sont les suivantes :
   SPECTRE DE MASSE: LC/MS Electrospray sur WATERS UPLC - SQD:
      Temps de rétention Tr (min) = 0,81 ;
      MH+ = 249+

### Exemple 55:

### N-(6-{[6-(4-fluorophényl)[1,2,4]triazolo[4,3-b]pyridazin-3-yl]sulfanyl}-1,3-benzothiazol-2-yl)morpholine-4-carboxamide

a) Le N-(6-{[6-(4-fluorophényl)[1,2,4]triazolo[4,3-b]pyridazin-3-yl]sulfanyl}-1,3-benzothiazol-2-yl)morpholine-4-carboxamide peut être préparé selon la méthode décrite à l'exemple 17a mais à partir de 640mg de thiocyanate de 2-[(morpholin-4-yl-carbonyl)amino]-1,3-benzothiazol-6-yle, de 20cm³ d'éthanol dégazé, de 10mg de dihydrogénophosphate de potassium dans 0,5cm³ d'eau, de 926mg de DL-dithiothréitol et de 496mg de 3-chloro-6-(4-fluorophényl)[1,2,4]triazolo[4,3-b]pyridazine. On obtient ainsi 133mg de N-(6-{[6-(4-fluorophényl)[1,2,4]triazolo[4,3-b]pyridazin-3-yl]sulfanyl}-1,3-benzothiazol-2-yl)morpholine-4-carboxamide sous forme d'un solide beige dont les caractéristiques sont les suivantes :
   POINT DE FUSION : >255°C (Büchi B-545)
   SPECTRE DE MASSE: LC/MS Electrospray sur WATERS UPLC - SQD: MH+ = 508+ ; MH- = 506-
   SPECTRE RMN 1 H (400 MHz, DMSO-d6) δ ppm : 3.49 - 3.63 (m, 8 H) 7.41 (t, *J*=9.0 Hz, 2 H) 7.52 - 7.56 (m large, 2 H) 8.02 (d, *J*=9.8 Hz, 1 H) 8.08 - 8.15 (m masqué, 1 H) 8.11 (dd, *J*=9.0, 5.3 Hz, 2 H) 8.50 (d, *J*=9.8 Hz, 1 H) 11.52 (m étalé, 1 H)
b) le thiocyanate de 2-[(morpholin-4-ylcarbonyl)amino]-1,3-benzothiazol-6-yle peut être préparé selon la méthode décrite à l'exemple 9c mais à partir de 2,29g de (6-thiocyanato-1,3-benzothiazol-2-yl)carbamate de phényle ester dans 70 cm³ de tétrahydrofurane et 0,6cm³ de morpholine, après 4h à 50°C. On obtient ainsi 2,11g de thiocyanate de 2-[(morpholin-4-ylcarbonyl)amino]-1,3-benzothiazol-6-yle sous forme d'un solide blanc dont les caractéristiques sont les suivantes :
   SPECTRE DE MASSE: LC/MS Electrospray sur Waters ZQ :
      Temps de rétention Tr (min) = 3,42 ;
      [M+H]+ : m/z 321 ; [M-H]- : m/z 319

### Exemple 56:

### 6-{[6-(2-fluorophényl)[1,2,4]triazolo[4,3-b]pyridazin-3-yl]sulfanyl}-1,3-benzothiazol-2-amine

a) Le 6-{[6-(2-fluorophényl)[1,2,4]triazolo[4,3-b]pyridazin-3-yl]sulfanyl}-1,3-benzothiazol-2-amine peut être préparé selon la méthode décrite à l'exemple 17a mais à partir de 167mg de thiocyanate de 2-amino-1,3-benzothiazol-6-yle (commerciale), d'un mélange de 5cm³ d'éthanol et de 5cm³ de tétrahydrofuranne dégazé, de 3,71 mg de dihydrogénophosphate de potassium dans 0,1cm³ d'eau, de 372mg de DL-dithiothréitol et de 200mg de 3-chloro-6-(2-fluorophényl)[1,2,4]triazolo[4,3-b]pyridazine. On obtient ainsi 169mg de 6-{[6-(2-fluorophényl)[1,2,4]triazolo[4,3-b]pyridazin-3-yl]sulfanyl}-1,3-benzothiazol-2-amine sous forme d'une poudre blanche dont les caractéristiques sont les suivantes :
   POINT DE FUSION : >255°C (Büchi B-545)
   SPECTRE DE MASSE: LC/MS Electrospray sur WATERS UPLC - SQD: MH+ = 395+ ; MH- = 393-
   SPECTRE RMN 1 H (300 MHz, DMSO-d6) δ ppm : 7.31 (d, *J*=8.5 Hz, 1 H) 7.36 - 7.50 (m, 3 H) 7.61 - 7.76 (m, 5 H) 7.95 (d, *J*=2.0 Hz, 1 H) 8.49 (d, *J*=9.8 Hz, 1 H)
b) Le 3-chloro-6-(2-fluorophényl)[1,2,4]triazolo[4,3-b]pyridazine peut être préparé selon la méthode décrite à l'exemple 17c mais à partir de 820mg d'acide 2-fluorophényle boronique, de 1,84g d'hydroxyde de barium octahydrate, de 0,43g de [1,1'-bis (diphénylphosphino)ferrocène]di-chloropalladium (II) en complexe avec du dichlorométhane (1:1) et 1 g de 3,6-dichloro[1,2,4]triazolo[4,3-b]pyridazine commerciale dans 8cm³ de N, N-diméthylformamide dégazé et 1,96cm³ d'eau, après 4,5h à 80°C. On obtient ainsi 416mg de 3-chloro-6-(2-fluorophényl)[1,2,4]triazolo[4,3-b]pyridazine sous forme de cristaux beige dont les caractéristiques sont les suivantes :
   SPECTRE DE MASSE: LC/MS Electrospray sur WATERS UPLC - SQD:
      Temps de rétention Tr (min) = 0,77 ;
      MH+ = 249+

### Exemple 60:

### N-(6-{[6-(4-fluorophényl)[1,2,4]triazolo[4,3-b]pyridazin-3-yl]sulfanyl}-1,3-benzothiazol-2-yl)cyclopropanecarboxamide

a) Le N-(6-{[6-(4-fluorophényl)[1,2,4]triazolo[4,3-b]pyridazin-3-yl]sulfanyl}-1,3-benzothiazol-2-yl)cyclopropanecarboxamide peut etre préparé de manière similaire à la méthode décrite à l'exemple 18b mais à partir de 591mg de 6-{[6-(4-fluorophényl)[1,2,4]triazolo[4,3-b]pyridazin-3-yl]sulfanyl}-1,3-benzothiazol-2-amine dans 3cm³ de pyridine avec 0,46cm³ de chlorure de l'acide cyclopropane carboxylique. Après 5h de réaction, à 20°C on ajoute de l'eau et on essore le précipité, que l'on lave à l'eau et à l'ether. Après chromatographie du précipité sur silice, on obtient 201 mg de N-(6-{[6-(4-fluorophényl)[1,2,4]triazolo[4,3-b]pyridazin-3-yl]sulfanyl}-1,3-benzothiazol-2-yl)cyclopropanecarboxamide sous forme de poudre blanche dont les caractéristiques sont les suivantes :
   POINT DE FUSION : >255°C (Büchi B-545)
   SPECTRE DE MASSE: LC/MS Electrospray sur WATERS UPLC - SQD: MH+ = 463+ ; MH- = 461-
   SPECTRE RMN 1 H (400 MHz, DMSO-d6) δ ppm : 0.92 - 0.98 (m, 4 H) 1.98 (m, 1 H) 7.40 (t, *J*=8.8 Hz, 2 H) 7.57 (dd, *J*=8.6, 2.0 Hz, 1 H) 7.71 (d, *J*=8.6 Hz, 1 H) 8.03 (d, *J*=9.8 Hz, 1 H) 8.10 (dd, *J*=8.8, 5.4 Hz, 2 H) 8.21 (d, *J*=2.0 Hz, 1 H) 8.51 (d, *J*=9.8 Hz, 1 H) 12.67 (m étalé, 1 H)

### Exemple 64:

### 1-(6-{[6-(3-fluorophényl)[1,2,4]triazolo[4,3-b]pyridazin-3-yl]sulfanyl}-1,3-benzothiazol-2-yl)-3-[2-(morpholin-4-yl)éthyl]urée

Le 1-(6-{[6-(3-fluorophényl)[1,2,4]triazolo[4,3-b]pyridazin-3-yl]sulfanyl}-1,3-benzothiazol-2-yl)-3-[2-(morpholin-4-yl)éthyl]urée peut être préparé selon la méthode décrite à l'exemple 16a mais à partir de 670mg de 3-chloro-6-(3-fluorophényl)[1,2,4]triazolo[4,3-b]pyridazine, de 13,4cm³ d'éthanol dégazé, de 127mg de borohydrure de sodium et de 1,14g de 1-[2-(morpholin-4-yl)éthyl]-3-(6-sulfanyl-1,3-benzothiazol-2-yl)urée. On obtient ainsi 135mg de 1-(6-{[6-(3-fluorophényl)[1,2,4]triazolo[4,3-b]pyridazin-3-yl]sulfanyl}-1,3-benzothiazol-2-yl)-3-[2-(morpholin-4-yl)éthyl]urée sous forme de poudre beige dont les caractéristiques sont les suivantes :
POINT DE FUSION : 251°C (Büchi B-545)
SPECTRE DE MASSE: LC/MS Electrospray sur WATERS UPLC - SQD: MH+ = 551 + ; MH- = 549-
SPECTRE RMN 1 H (300 MHz, DMSO-d6) δ ppm : 2.37 - 2.45 (m, 6 H) 3.23 - 3.29 (m partiellement masqué, 2 H) 3.59 (m, 4 H) 6.79 (m large, 1 H) 7.44 (m, 1 H) 7.51 (dd, J=8.5, 2.0 Hz, 1 H) 7.56 - 7.66 (m, 2 H) 7.79 (dm, J=10.4 Hz, 1 H) 7.89 (dm, J=8.0 Hz, 1 H) 8.05 (d, J=9.9 Hz, 1 H) 8.16 (d, J=2.0 Hz, 1 H) 8.53 (d, J=9.9 Hz, 1 H) 10.96 (m étalé; 1 H)

### Exemple 65:

### N-(6-{[6-(3-fluorophényl)[1,2,4]triazolo[4,3-b]pyridazin-3-yl]sulfanyl}-1,3-benzothiazol-2-yl)acétamide

Le N-(6-{[6-(3-fluorophényl)[1,2,4]triazolo[4,3-b]pyridazin-3-yl]sulfanyl}-1,3-benzothiazol-2-yl)acétamide peut être préparé selon la méthode décrite à l'exemple 18b mais à partir de 100g de 6-{[6-(3-fluorophényl)[1,2,4]triazolo[4,3-b]pyridazin-3-yl]sulfanyl}-1,3-benzothiazol-2-amine dans 5cm³ de pyridine et de 0,318cm³ d'anhydride acétique à 50°C pendant 4h. On obtient ainsi 54mg de N-(6-{[6-(3-fluorophényl)[1,2,4]triazolo[4,3-b]pyridazin-3-yl]sulfanyl}-1,3-benzothiazol-2-yl)acétamide sous forme d'une poudre blanche dont les caractéristiques sont les suivantes :
POINT DE FUSION > 255°C (Büchi B-545)
SPECTRE DE MASSE: LC/MS Electrospray sur WATERS UPLC - SQD: MH+ = 437+ ; MH- = 435-
SPECTRE RMN 1H (300 MHz, DMSO-d6) δ ppm : 2.18 (s, 3 H) 7.43 (m, 1 H) 7.52 - 7.65 (m, 2 H) 7.70 (d, J=8.5 Hz, 1 H) 7.77 (ddd, J=10.3, 2.7, 1.6 Hz, 1 H) 7.88 (m, 1 H) 8.05 (d, J=9.8 Hz, 1 H) 8.23 (d large, J=2.0 Hz, 1 H) 8.54 (d, J=9.8 Hz, 1 H) 12.38 (m étalé, 1 H)

### Exemple 66:

### N-(6-{[6-(4-fluorophényl)[1,2,4]triazolo[4,3-b]pyridazin-3-yl]sulfanyl}-1,3-benzothiazol-2-yl)-2-méthylpropanamide

a) Le N-(6-{[6-(4-fluorophényl)[1,2,4]triazolo[4,3-b]pyridazin-3-yl]sulfanyl}-1,3-benzothiazol-2-yl)-2-méthylpropanamide peut être préparé de manière similaire à la méthode décrite à l'exemple 18b mais à partir de 560mg de 6-{[6-(4-fluorophényl)[1,2,4]triazolo[4,3-b]pyridazin-3-yl]sulfanyl}-1,3-benzothiazol-2-amine et de 0,212cm³ de chlorure de 2-méthylpropanoyle dans 3cm³ de pyridine après 24h à 20°C. Ensuite, on additionne de l'eau et agite 5min puis le précipité est essoré, lavé à l'eau et séché sous vide. On obtient ainsi, après purification sur silice, 305mg de N-(6-{[6-(4-fluorophényl)[1,2,4]triazolo[4,3-b]pyridazin-3-yl]sulfanyl}-1,3-benzothiazol-2-yl)-2-méthylpropanamide sous forme de solide jaune pâle dont les caractéristiques sont les suivantes :
   POINT DE FUSION > 255°C (Büchi B-545)
   SPECTRE DE MASSE: LC/MS Electrospray sur WATERS UPLC - SQD: MH+ = 465 ; MH- = 463
   SPECTRE RMN 1H (400 MHz, DMSO-d6) δ ppm : 1.14 (d, J=6.8 Hz, 6 H) 2.77 (m, 1 H) 7.40 (t, J=9.0 Hz, 2 H) 7.57 (dd, J=8.6, 2.0 Hz, 1 H) 7.71 (d, J=8.6 Hz, 1 H) 8.03 (d, J=9.8 Hz, 1 H) 8.10 (dd, J=9.0, 5.4 Hz, 2 H) 8.23 (d, J=2.0 Hz, 1 H) 8.52 (d, J=9.8 Hz, 1 H) 12.24 (s large, 1 H)

### Exemple 67:

### N-(6-{[6-(3-fluorophényl)[1,2,4]triazolo[4,3-b]pyridazin-3-yl]sulfanyl}-1,3-benzothiazol-2-yl)cyclopropanecarboxamide

a) Le N-(6-{[6-(3-fluorophényl)[1,2,4]triazolo[4,3-b]pyridazin-3-yl]sulfanyl}-1,3-benzothiazol-2-yl)cyclopropanecarboxamide peut être préparé de manière similaire à la méthode décrite à l'exemple 18b mais à partir de 100mg de 6-{[6-(3-fluorophényl)[1,2,4]triazolo[4,3-b]pyridazin-3-yl]sulfanyl}-1,3-benzothiazol-2-amine dans 1cm³ de pyridine avec 0,028cm³ de chlorure de l'acide cyclopropane carboxylique. Après 3h de réaction, à 20°C on ajoute de l'eau et on essore le précipité, que l'on lave à l'eau et à l'ether. Après chromatographie du précipité sur cartouche de silice Biotage, on obtient 51mg de N-(6-{[6-(3-fluorophényl)[1,2,4]triazolo[4,3-b]pyridazin-3-yl]sulfanyl}-1,3-benzothiazol-2-yl)cyclopropanecarboxamide sous forme de poudre blanche dont les caractéristiques sont les suivantes :
   POINT DE FUSION : > 255°C (Büchi B-545)
   SPECTRE DE MASSE: LC/MS Electrospray sur WATERS UPLC - SQD: MH+ = 463+ ; MH- = 461-
   SPECTRE RMN 1H (300 MHz, DMSO-d6) δ ppm : 0.84 - 1.01 (m, 4 H) 1.98 (m, 1 H) 7.43 (tdd, J=8.5, 2.6, 1.0 Hz, 1 H) 7.51 - 7.65 (m, 2 H) 7.70 (d, J=8.5 Hz, 1 H) 7.77 (ddd, J=10.4, 2.6, 1.7 Hz, 1 H) 7.88 (ddd, J=7.8, 1.7, 1.0 Hz, 1 H) 8.05 (d, J=9.8 Hz, 1 H) 8.22 (d large, J=2.0 Hz, 1 H) 8.54 (d, J=9.8 Hz, 1 H) 12.68 (m étalé, 1 H)

### Exemple 69: Composition pharmaceutique

On a préparé des comprimés répondant à la formule suivante:
Produit de l'exemple 1 0,2 g
Excipient pour un comprimé terminé à 1 g
   (détail de l'excipient : lactose, talc, amidon, stéarate de magnésium).

### Exemple 70: Composition pharmaceutique

On a préparé des comprimés répondant à la formule suivante :
Produit de l'exemple 4 0,2 g
Excipient pour un comprimé terminé à 1 g
   (détail de l'excipient : lactose, talc, amidon, stéarate de magnésium).

Les exemples 1 et 4 sont pris à titre d'exemples de préparation pharmaceutique, cette préparation pouvant être réalisée si désiré avec d'autres produits en exemples dans la présente demande.

### Partie pharmacologique :

### Protocoles expérimentaux

### I) Expression et Purification de MET, domaine cytoplasmique

### Expression en Baculovirus:

L'ADN recombinant His-Tev-MET (956-1390) en pFastBac(Invitrogen) est transfecté dans des cellules d'insectes et après plusieurs étapes d'amplification virale, le stock de baculovirus final est testé pour l'expression de la protéine d'intérêt.

Après infection pendant 72h à 27°C avec le virus recombinant, les cultures de cellules SF21 sont récoltées par centrifugation et les culots cellulaires sont stockés à -80°C.

### Purification :

Les culots cellulaires sont remis en suspension dans le tampon de lyse (tampon A [50 mM HEPES, pH 7.5, 250 mM NaCl, Glycérol 10%, TECP 1 mM]; + cocktail d'inhibiteurs de protéases Roche Diagnostics sans EDTA, réf 1873580), agités à 4°C jusqu'à homogénéité, puis lysés mécaniquement en utilisant un appareil de type « Dounce ».

Après centrifugation, le surnageant de lyse est incubé 2h à 4°C avec de la résine Nickel Chelate (His-Trap 6 Fast Flow ^{™}, GE HealthCare). Après lavage avec 20 volumes de Tp A, la suspension est packée dans une colonne, et les protéines sont éluées par un gradient de tampon B (TpA + 290 mM imidazole).

Les fractions contenant la protéine d'intérêt au vu de l'analyse électrophorétique (SDS PAGE) sont rassemblées, concentrés par Ultrafiltration (cut-off 10kDa) et injectées sur une colonne de chromatographie d'exclusion (Superdex ^{™} 200 , GE HealthCare) équilibrée en tampon A. Après clivage enzymatique du tag Histidine, la protéine est réinjectée sur une nouvelle colonne de chromatographie IMAC Nickel Chelate (His-Trap 6 Fast Flow ^{™}, GE HealthCare) équilibrée en Tampon A. Les fractions éluées par un gradient de tampon B et contenant la protéine d'intérêt après électrophorèse (SDS PAGE), sont finalement rassemblées et conservées à -80°C.

Pour la production de protéine autophosphorylée, les fractions précédentes sont incubées 1h à température ambiante après ajout d'ATP 2mM, MgCl2 2mM, et Na3VO4 4mM. Après arrêt de la réaction avec 5mM d'EDTA, le mélange réactionnel est injecté sur une colonne de dessalage HiPrep (GE HealthCare) préalablement équilibré en tampon A + Na3VO4 4mM, les fractions contenant la protéine d'intérêt (analyse SDS PAGE) sont rassemblées et stockées à -80°C. Le taux de phosphorylation est vérifié par spectrométrie de masse (LC-MS), et par peptide mapping.

### II) Tests A et B

### A) Test A : Essai HTRF MET en format 96 puits

Dans un volume final de 50µl de réaction enzymatique, MET 5nM final est incubé en présence de la molécule à tester (pour une gamme concentration finale de 0,17 nM à 10 µM, DMSO 3% final) en tampon MOPS 10mM pH 7.4 , DTT 1 mM, Tween 20 0.01 %. La réaction est initiée par la solution substrats pour obtenir les concentrations finales de poly-(GAT) 1 µg/ml, ATP 10µM et MgCl2 5mM. Après une incubation de 10 min à température ambiante, la réaction est arrêtée par un mix de 30 µl pour obtenir une solution finale d' Hepes 50mM pH 7.5, fluorure de potassium 500mM, BSA 0,1% et EDTA 133mM en présence de 80ng Streptavidine 61SAXLB Cis-Bio Int. et 18ng anti-Phosphotyrosine Mab PT66-Europium Cryptate par puits. Après 2 heures d'incubation à température ambiante, la lecture est faite à 2 longueur d' ondes 620nm et 665 nm sur un lecteur pour la technique TRACE / HTRF et le % d'inhibition est calculé d'après les ratios 665/620.

Les résultats obtenus par ce test A pour les produits de formule (I) en exemples dans la partie expérimentale sont tels que IC50 inférieure à 500nM et notamment à 100nM.

### B) Test B : Inhibition de l'autophosphorylation de MET ; technique ELISA (pppY1230,1234,1235)

### a) Lysats cellulaires : Ensemencer les cellules MKN45 en plaque 96 puits (Cell coat BD polylysine) à 20 000 cellules/puits sous 200µl en milieu RPMI + 10%SVF + 1 % L-glutamine. Laisser adhérer 24h à l'incubateur.

Les cellules sont traitées le lendemain de l'ensemencement avec les produits à 6 concentrations en duplicate pendant 1 h. Au moins 3 puits contrôles sont traités avec la même quantité de DMSO finale.

Dilution des produits : Stock à 10mM dans le DMSO pur - Gamme de 10mM à 30µM avec un pas de 3 en DMSO pur - Dilutions intermédiaires au 1/50 dans du milieu de culture puis prélèvement de 10µl ajoutés directement aux cellules (200µl) : gamme finale de 10000 à 30nM.

A la fin de l'incubation, éliminer délicatement le surnageant et faire un rinçage avec 200µl de PBS. Puis mettre 100µl de tampon de lyse directement dans les puits sur la glace et incuber à 4°C pendant 30minutes. Tampon de lyse : 10m Tris, HCl pH7.4, 100mM NaCl, 1mM EDTA, 1mM EGTA, 1% Triton X-100, 10% glycerol, 0.1% SDS, 0.5% deoxycholate, 20mM NaF, 2mM Na3VO4, 1 mM PMSF et cocktail anti protéases.

Les 100µl de lysats sont transférés dans une plaque en polypropylène fond en V et l'ELISA est réalisé de suite ou la plaque est congelée à -80°C.

### b) ELISA PhosphoMET BioSource Kit KHO0281

Dans chaque puits de la plaque du kit, ajouter 70µl de tampon de dilution du kit + 30µL de lysat cellulaire ou 30µl de tampon de lyse pour les blancs. Incuber pendant 2h sous agitation douce à température ambiante.

Rincer 4 fois les puits avec 400µl de tampon de lavage du kit. Incuber avec 100µl d'Anticorps anti-phospho MET pendant 1 h à température ambiante.

Rincer 4 fois les puits avec 400µl de tampon de lavage du kit. Incuber avec 100 µl d'Anticorps anti-lapin HRP pendant 30 minutes à température ambiante (sauf pour les puits chromogen seul).

Rincer 4 fois les puits avec 400µl de tampon de lavage du kit. Mettre 100µL de chromogen et incuber 30minutes dans le noir à température ambiante.

Arrêter la réaction avec 100µl de solution stop. Lire sans tarder à 450nM 0,1 seconde au Wallac Victor plate reader.

### C) Test C : Mesure de la prolifération cellulaire par pulse de 14C-thymidine

Les cellules sont ensemencées dans des plaques Cytostar 96 puits sous 180µl pendant 4 heures à 37°C et 5% CO2 : Les cellules HCT116 à raison de 2500 cellules par puits dans du milieu DMEM + 10% sérum de veau foetal + 1 % de L-Glutamine et les cellules MKN45 à raison de 7500 cellules par puits dans du milieu RPMI + 10% sérum de veau foetal + 1% de L-Glutamine. Après ces 4 heures d'incubation, les produits sont ajoutés sous 10µl en solution 20 fois concentrée selon la méthode de dilution citée pour l'ELISA. Les produits sont testés à 10 concentrations en duplicate de 10000nM à 0,3nM avec un pas de 3.

Après 72h de traitement, ajouter 10µl de 14C-thymidine à 10µCi/ml pour obtenir 0,1µCi par puits. L'incorporation de 14C-thymidine est mesurée sur un Micro-Beta (Perkin-Elmer) après 24 heures de pulse et 96h de traitement.

Toutes les étapes de l'essai sont automatisées sur les stations BIOMEK 2000 ou TECAN.

Les résultats obtenus par ce test B pour les produits de formule (I) en exemples dans la partie expérimentale sont tels que IC50 inférieure à 10microM et notamment à 1microM.

Les résultats obtenus pour les produits en exemples dans la partie expérimentale sont donnés dans le tableau de résultats pharmacologiques ci-après, comme suit :
pour le test A, le signe + correspond à inférieur à 500nM et le signe ++ correspond à inférieur à 100nM.
pour le test B le signe + correspond à inférieur à 500nM et le signe ++ correspond à inférieur à 100nM.
pour le test C le signe + correspond à inférieur à 10microM et le signe ++ correspond à inférieur à 1microM.

**Tableau de résultats pharmacologiques :**

| **numéro ex** | **test A** | **test B** | **test C** |
|---|---|---|---|
| 1 | ++ | ++ | ++ |
| 2 | ++ | + | ++ |
| 3 | ++ | ++ | ++ |
| 9 | ++ | ++ | ++ |
| 10 | ++ | ++ | ++ |
| 11 | ++ | ++ | ++ |
| 12 | ++ | ++ | ++ |
| 14 | ++ | ++ | ++ |
| 15 | ++ | ++ | ++ |
| 17 | ++ | ++ | ++ |
| 26 | ++ | ++ | ++ |
| 30 | ++^{.} | ++ | ++ |
| 33 | + | ++ | ++ |
| 41 | ++ | ++ | ++ |
| 42 | ++ | ++ | ++ |
| 53 | ++ | ++ | ++ |
| 55 | ++ | + | ++ |
| 56 | ++ | + | ++ |
| 60 | ++ | ++ | ++ |
| 64 | ++ | ++ | ++ |
| 65 | ++ | ++ | ++ |
| 66 | ++ | + | ++ |
| 67 | ++ | ++ | ++ |

## Revendications

1. Produits de formule (I"b) : 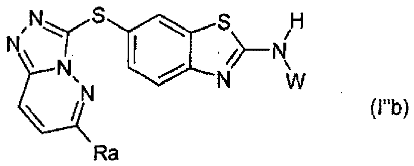 dans laquelle
- Ra représente un atome d'hydrogène ou bien un atome de chlore ou bien le radical : 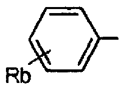 avec Rb représente un atome d'halogène ou un radical S-hétéroaryle éventuellement substitué par un radical choisi parmi les atomes d'halogène et les radicaux hydroxyle, alkyle et alcoxy renfermant de 1 à 4 atomes de carbone, NH2, NHalk et N(alk)2 ;
W représente un atome d'hydrogène ou un radical alkyle ou le radical COR dans lequel R représente :
- un radical alkyle éventuellement substitué par OCH3 ou NR3R4 ;
- un radical cycloalkyle
- un radical alcoxy éventuellement substitué par OCH3 ou NR3R4, càd un radical O-(CH2)n- OCH3 ou un radical O-(CH2)n-NR3R4 ; un radical O-phényle ou O-(CH2)n-phényle, avec n représente un entier de 1 à 2 ;
- ou le radical NR1R2, dans lequel R1 et R2 sont tels que l'un de R1 et R2 représente un atome d'hydrogène, un radical cycloalkyle où un radical alkyle et l'autre de R1 et R2 représente un radical alkyle éventuellement substitué par NR3R4, ou bien R1 et R2 forment avec l'atome d'azote auquel ils sont liés un radical cyclique renfermant de 3 à 10 chaînons et renfermant éventuellement un ou plusieurs autres hétéroatomes choisi(s) parmi O, S, N et NH;
avec NR3R4 tel que R3 et R4, identiques ou différents, représentent un atome d'hydrogène ou un radical alkyle ou bien R3 et R4 forment avec l'atome d'azote auquel ils sont liés un radical cyclique renfermant éventuellement un ou plusieurs autres hétéroatomes choisi(s) parmi O, S, N et NH, ce radical y compris l'éventuel NH qu'il contient étant éventuellement substitué par un ou plusieurs radicaux choisis parmi les radicaux alkyle ;
avec le terme radical alkyle ou Alk désignant un radical alkyle renfermant de 1 à 6 atomes de carbone,
avec le terme radical cycloalkyle désignant un radical carbocyclique saturé renfermant 3 à 10 atomes de carbone,
avec le terme alcoxy désignant un radical renfermant de 1 à 4 atomes de carbone,
avec le terme hétéroaryle désignant un radical monocyclique ou bicyclique renfermant de 5 à 12 chaînons,
lesdits produits de formule (I"b) étant sous toutes les formes isomères possibles racémiques, énantiomères et diastéréoisomères, ainsi que les sels d'addition avec les acides minéraux et organiques ou avec les bases minérales et organiques desdits produits de formule (I"b).

2. Produits de formule (I"b) tels que définis à la revendication 1, répondant aux formules suivantes :
- (6-{[6-(4-fluorophényl)[1,2,4]triazolo[4,3-b]pyridazin-3-yl]sulfanyl}-1,3-benzothiazol-2-yl)carbamate de méthyle
- (6-{[6-(4-fluorophényl)[1,2,4]triazolo[4,3-b]pyridazin-3-yl]sulfanyl}-1,3-benzothiazol-2-yl)carbamate de 1,1-diméthyléthyle
- 6-{[6-(4-fluorophényl)[1,2,4]triazolo[4,3-b]pyridazin-3-yl]sulfanyl}-1,3-benzothiazol-2-amine
- 1-(2-morpholin-4-yléthyl)-3-[6-([1,2,4]triazolo[4,3-b]pyridazin-3-ylsulfanyl)-1,3-benzothiazol-2-yl]urée
- 1-(6-{[6-(4-fluorophényl)[1,2,4]triazolo[4,3-b]pyridazin-3-yl]sulfanyl}-1,3-benzothiazol-2-yl)-3-(2-morpholin-4-yléthyl)urée
- 1-{2-[(2R,6S)-2,6-diméthylmorpholin-4-yl]éthyl}-3-(6-{[6-(4-fluorophényl)[1,2,4]triazolo[4,3-b]pyridazin-3-yl]sulfanyl}-1,3-benzothiazol-2-yl)urée
- (6-{[6-(4-fluorophényl)[1,2,4]triazolo[4,3-b]pyridazin-3-yl]sulfanyl}-1,3-benzothiazol-2-yl)carbamate de 2-morpholin-4-yléthyle
- 1-(6-{[6-(4-fluorophényl)[1,2,4]triazolo[4,3-b]pyridazin-3-yl]sulfanyl}-1,3-benzothiazol-2-yl)-3-[2-(4-méthylpipérazin-1-yl)éthyl]urée
- N-(6-{[6-(4-fluorophényl)[1,2,4]triazolo[4,3-b]pyridazin-3-yl]sulfanyl}-1,3-benzothiazol-2-yl)-4-morpholin-4-ylbutanamide
- 1-[2-(diéthylamino)éthyl]-3-(6-{[6-(4-fluorophényl)[1,2,4]triazolo[4,3-b]pyridazin-3-yl]sulfanyl}-1,3-benzothiazol-2-yl)urée
- 6-{[6-(4-fluorophényl)[1,2,4]triazolo[4,3-b]pyridazin-3-yl]sulfanyl}-1,3-benzothiazol-2-yl)carbamate de phényle
- N-(6-{[6-(4-fluorophényl)[1,2,4]triazolo[4,3-b]pyridazin-3-yl]sulfanyl}-1,3-benzothiazol-2-yl)acétamide
- 6-{[6-(3-fluorophényl)[1,2,4]triazolo[4,3-b]pyridazin-3-yl]sulfanyl}-1,3-benzothiazol-2-amine
- N-(6-{[6-(4-fluorophényl)[1,2,4]triazolo[4,3-b]pyridazin-3-yl]sulfanyl}-1,3-benzothiazol-2-yl)morpholine-4-carboxamide
- 6-{[6-(2-fluorophényl)[1,2,4]triazolo[4,3-b]pyridazin-3-yl]sulfanyl}-1,3-benzothiazol-2-amine
- N-(6-{[6-(4-fluorophényl)[1,2,4]triazolo[4,3-b]pyridazin-3-yl]sulfanyl}-1,3-benzathiazol-2-yl)cyclopropanecarboxamide
- 1-(6-{[6-(3-fluorophényl)[1,2,4]triazolo[4,3-b]pyridazin-3-yl]sulfanyl}-1,3-benzothiazol-2-yl)-3-[2-(morpholin-4-yl)éthyl]urée
- N-(6-{[6-(3-fluorophényl)[1,2,4]triazolo[4,3-b]pyridazin-3-yl]sulfanyl}-1,3-benzothiazol-2-yl)acétamide;
- N-(6-{[6-(4-fluorophényl)[1,2,4]triazolo[4,3-b]pyridazin-3-yl]sulfanyl}-1,3-benzothiazol-2-yl)-2-méthylpropanamide;
- N-(6-{[6-(3-fluorophényl)[1,2,4]triazolo[4,3-b]pyridazin-3-yl]sulfanyl}-1,3-benzothiazol-2-yl)cyclopropanecarboxamide ; ainsi que les sels d'addition avec les acides minéraux et organiques ou avec les bases minérales et organiques pharmaceutiquement acceptables desdits produits de formule (I"b).

3. Procédé de préparation des produits de formule (I"b) tels que définis à la revendication 1, **caractérisé par** le schéma suivant 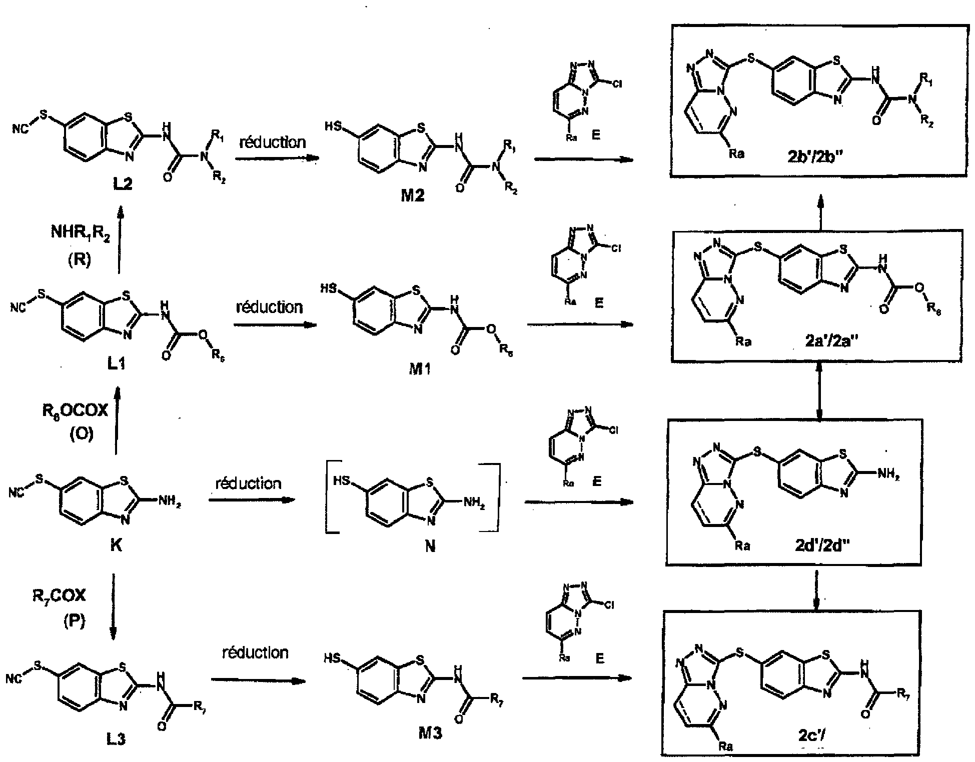 dans lequel les substituants, Ra, R1 et R2 ont les significations indiquées à la revendication 1, le substituant R6 représente un radical alkyle éventuellement substitué par un groupe NR3R4 ou OCH3, un radical phényle, -(CH₂)n-phényle, et n représentant un entier de 1 à 2, le substituant R7 représente un radical cycloalkyle ou alkyle éventuellement substitué par un radical NR3R4 ou OCH3, dans lesquels R3 et R4 ont les significations indiquées à la revendication 1 et n représente un entier de 1 à 2 ;
dans les composés de formules 2a", 2b", 2c" et 2d", représente une double liaison, étant entendu que
- la réduction des composés de formules (L1), (L2), (L3) respectivement en composés (M1), (M2) et (M3) est obtenue avec du DL-dithiotréitol, en présence de dihydrogénocarbonate de sodium, dans un solvant et à une température voisine de 80 °C,
- la réduction du composé K en composé (N) est obtenue avec du borohydrure de sodium dans un solvant, en présence d'une base et à une température voisine de 95 °C ou comprise entre 20 °C et 95 °C,
étant entendu que l'on peut soumettre, si désiré et si nécessaire, des produits intermédiaires ou des produits de formule (I"b) ainsi obtenus par les procédés indiqués ci-dessus, pour obtenir d'autres intermédiaires ou d'autres produits de formule (I"b), à une ou plusieurs réactions de transformations connues de l'homme du métier, en particulier :
a) une réaction d'estérification de fonction acide,
b) une réaction de saponification de fonction ester en fonction acide,
c) une réaction de réduction de la fonction carboxy libre ou estérifié en fonction alcool,
d) une réaction de transformation de fonction alcoxy en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formule (I"b) ainsi obtenus étant sous toutes les formes isomères possibles racémiques, énantiomères et diastéréoisomères.

4. A titre de médicaments, les produits de formule (I"b) telle que définie à l'une des revendications 1 et 2, ainsi que les sels d'addition avec les acides minéraux et organiques ou avec les bases minérales et organiques pharmaceutiquement acceptables desdits produits de formule (I"b).

5. Compositions pharmaceutiques contenant à titre de principe actif l'un au moins des produits de formule (I"b) tel que défini à l'une des revendications 1 et 2 ou un sel pharmaceutiquement acceptable de ce produit et un support pharmaceutiquement acceptable.

6. Utilisation d'un produit de formule (I"b) tel que défini à l'une des revendications 1 et 2, pour la préparation d'un médicament destiné au traitement ou à la prévention d'une maladie choisie dans le groupe suivant : troubles de la prolifération de vaisseaux sanguins, troubles fibrotiques, troubles de la prolifération de cellules 'mesangial', désordres métaboliques, allergies, asthmes, thromboses, maladies du système nerveux, rétinopathie, psoriasis, arthrite rhumatoïde, diabète, dégénérescence musculaire et cancers.

7. Utilisation d'un produit de formule (I"b) tel que défini à l'une des revendications 1 et 2 pour la préparation d'un médicament destiné au traitement de cancers.

8. Utilisation selon la revendication 7 destinée au traitement de tumeurs solides ou liquides.

9. Utilisation selon la revendication 7 ou 8 destinée au traitement de cancers résistant à des agents cytotoxiques.

10. Utilisation selon l'une ou plusieurs des revendications 7 à 9 destinée au traitement de tumeurs primaires et/ou de métastases en particulier dans les cancers gastriques, hépatiques, rénaux, ovariens, du colon, de la prostate, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas.

11. Utilisation des produits de formule (I"b) telle que définie aux revendications 1 et 2, pour la préparation de médicaments destinés à la chimiothérapie de cancers.

12. Utilisation des produits de formule (I"b) telle que définie aux revendications 1 et 2, pour la préparation de médicaments destinés à la chimiothérapie de cancers seul ou en en association.

13. A titre de produits industriels nouveaux, les intermédiaires de synthèse de formules E', M1, M2 et M3: 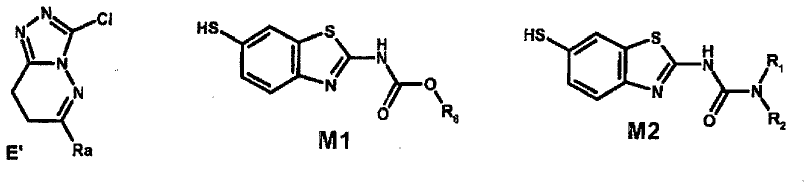 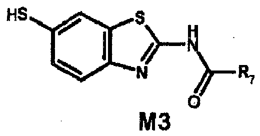 dans lesquels R6 représente un radical alkyle éventuellement substitué par un groupe NR3R4 ou OCH3, un radical phényle, -(CH2)n-phényle, et n représente un entier de 1 à 2, ; R7 représente un radical cycloalkyle ou alkyle éventuellement substitué par un radical NR3R4 ou OCH3 ; et Ra, R1, R2, R3 et R4 ont les significations indiquées à la revendication 1.

## Patentansprüche

1. Produkte der Formel (I''b): 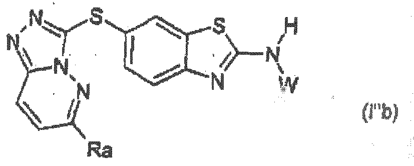 worin:
- Ra für ein Wasserstoffatom oder ein Chloratom oder den Rest: 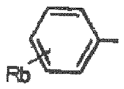 steht, wobei Rb für ein Halogenatom oder einen S-Heteroarylrest, der gegebenenfalls durch einen aus Halogenatomen und Hydroxyl-, Alkyl- und Alkoxyresten mit 1 bis 4 Kohlenstoffatomen, NH2, NHAlk und N(Alk)₂ ausgewählten Rest substituiert ist, steht;
W für ein Waserstoffatom oder einen Alkylrest oder den Rest COR steht, wobei R für
- einen Alkylrest, der gegebenenfalls durch OCH3 oder NR3R4 substituiert ist;
- einen Cycloalkylrest;
- einen Alkoxyrest, der gegebenenfalls durch OCH3 oder NR3R4 substituiert ist, d.h. einen O-(CH2)n-OCH3- oder O-(CH2)n-NR3R4-Rest; einen O-Phenyl- oder O-(CH2)n-Phenyl-Rest steht, wobei n für eine ganze Zahl von 1 bis 2 steht;
- oder den Rest NR1R2 steht, worin R1 und R2 so beschaffen sind, dass einer der Reste R1 und R2 für ein Wasserstoffatom, einen Cycloalkylrest oder einen Alkylrest steht und der andere der Reste R1 und R2 für einen gegebenenfalls durch NR3R4 substituierten Alkylrest steht, oder R1 und R2 mit dem Stickstoffatom, an das sie gebunden sind, einen cyclischen Rest mit 3 bis 10 Gliedern und gegebenenfalls einem oder mehreren weiteren Heteroatomen, die aus O, S, N und NH ausgewählt sind, bilden;
steht; wobei NR3R4 so beschaffen ist, dass R3 und R4 gleich oder verschieden sind und für ein Wasserstoffatom oder einen Alkylrest stehen oder R3 und R4 mit dem Stickstoffatom, an das sie gebunden sind, einen cyclischen Rest mit gegebenenfalls einem oder mehreren weiteren Heteroatomen, die aus O, S, N und NH ausgewählt sind, bilden, wobei dieser Rest einschließlich des eventuellen NH, das er enthält, gegebenenfalls durch einen oder mehrere Reste, die aus Alkylresten ausgewählt sind, substituiert ist; wobei der Begriff Alkyl- oder Alk-Rest für einen Alkylrest mit 1 bis 6 Kohlenstoffatomen steht, wobei der Begriff Cycloalkylrest für einen gesättigten carbocyclischen Rest mit 3 bis 10 Kohlenstoffatomen steht,
wobei der Begriff Alkoxy für einen Rest mit 1 bis 4 Kohlenstoffatomen steht,
wobei der Begriff Heteroaryl für einen monocyclischen oder bicyclischen Rest mit 5 bis 12 Gliedern steht,
wobei die Produkte der Formel (I''b) in allen möglichen racemischen, enantiomeren und diastereoisomeren isomeren Formen vorliegen, sowie die Additionssalze der Produkte der Formel (I''b) mit anorganischen und organischen Säuren oder mit anorganischen und organischen Basen.

2. Produkte der Formel (I''b) nach Anspruch 1, die den folgenden Formeln entsprechen:
- (6-{[6-(4-Fluorphenyl)[1,2,4]triazolo[4,3-b]-pyridazin-3-yl]sulfanyl}-1,3-benzothiazol-2-yl)carbamidsäuremethylester
- (6-{[6-(4-Fluorphenyl)[1,2,4]triazolo[4,3-b]-pyridazin-3-yl]sulfanyl}-1,3-benzothiazol-2-yl)carbamidsäure-1,1-dimethylethylester
- 6-{[6-(4-Fluorphenyl)[1,2,4]triazolo[4,3-b]-pyridazin-3-yl]sulfanyl}-1,3-benzothiazol-2-amin
- 1-(2-Morpholin-4-ylethyl)-3-[6-([1,2,4]-triazolo[4,3-b]pyridazin-3-ylsulfanyl)-1,3-benzothiazol-2-yl]harnstoff
- 1-(6-{[6-(4-Fluorphenyl)[1,2,4]triazolo[4,3-b]-pyridazin-3-yl]sulfanyl}-1,3-benzothiazol-2-yl)-3-(2-morpholin-4-ylethyl)harnstoff
- 1-{2-[(2R,6S)-2,6-Dimethylmorpholin-4-yl]-ethyl}-3-(6-{[6-(4-fluorphenyl)-[1,2,4]-triazolo[4,3-b]pyridazin-3-yl]sulfanyl}-1,3-benzothiazol-2-yl)harnstoff
- (6-{[6-(4-Fluorphenyl)[1,2,4]triazolo[4,3-b]-pyridazin-3-yl]sulfanyl}-1,3-benzothiazol-2-yl)carbamidsäure-2-morpholin-4-ylethylester
- 1-(6-{[6-(4-Fluorphenyl)[1,2,4]triazolo[4,3-b]-pyridazin-3-yl]sulfanyl}-1,3-benzothiazol-2-yl)-3-[2-(4-methylpiperazin-1-yl)ethyl]harnstoff
- N-(6-{[6-(4-Fluorphenyl)[1,2,4]triazolo[4,3-b]-pyridazin-3-yl]sulfanyl}-1,3-benzothiazol-2-yl)-4-morpholin-4-ylbutanamid
- 1-[2-(Diethylamino)ethyl]-3-(6-{[6-(4-fluorphenyl)[1,2,4]triazolo[4,3-b]pyridazin-3-yl]-sulfanyl}-1,3-benzothiazol-2-yl)harnstoff
- 6-{[6-(4-Fluorphenyl)[1,2,4]triazolo[4,3-b]-pyridazin-3-yl]sulfanyl}-1,3-benzothiazol-2-yl)carbamidsäurephenylester
- N-(6-{[6-(4-Fluorphenyl)[1,2,4]triazolo[4,3-b]-pyridazin-3-yl]sulfanyl}-1,3-benzothiazol-2-yl)acetamid
- 6-{[6-(3-Fluorphenyl)[1,2,4]triazolo[4,3-b]-pyridazin-3-yl]sulfanyl}-1,3-benzothiazol-2-amin
- N-(6-{[6-(4-Fluorphenyl)[1,2,4]triazolo[4,3-b]-pyridazin-3-yl]sulfanyl}-1,3-benzothiazol-2-yl)-morpholin-4-carboxamid
- 6-{[6-(2-Fluorphenyl)[1,2,4]triazolo[4,3-b]-pyridazin-3-yl]sulfanyl}-1,3-benzothiazol-2-amin
- N-(6-{[6-(4-Fluorphenyl)[1,2,4]triazolo[4,3-b]-pyridazin-3-yl]sulfanyl}-1,3-benzothiazol-2-yl)cyclopropancarboxamid
- 1-(6-{[6-(3-Fluorphenyl)[1,2,4]triazolo[4,3-b]-pyridazin-3-yl]sulfanyl}-1,3-benzothiazol-2-yl)-3-[2-(morpholin-4-yl)ethyl]harnstoff
- N-(6-{[6-(3-Fluorphenyl)[1,2,4]triazolo[4,3-b]-pyridazin-3-yl]sulfanyl}-1,3-benzothiazol-2-yl)acetamid
- N-(6-{[6-(4-Fluorphenyl)[1,2,4]triazolo[4,3-b]-pyridazin-3-yl]sulfanyl}-1,3-benzothiazol-2-yl)-2-methylpropanamid
- N-(6-{[6-(3-Fluorphenyl)[1,2,4]triazolo[4,3-b]-pyridazin-3-yl]sulfanyl}-1,3-benzothiazol-2-yl)cyclopropancarboxamid;
sowie die Additionssalze der Produkte der Formel (I''b) mit anorganischen und organischen Säuren oder mit anorganischen und organischen Basen, die pharmazeutisch unbedenklich sind.

3. Verfahren zur Herstellung der Produkte der Formel (I''b) gemäß Anspruch 1, **gekennzeichnet durch** das folgende Schema: 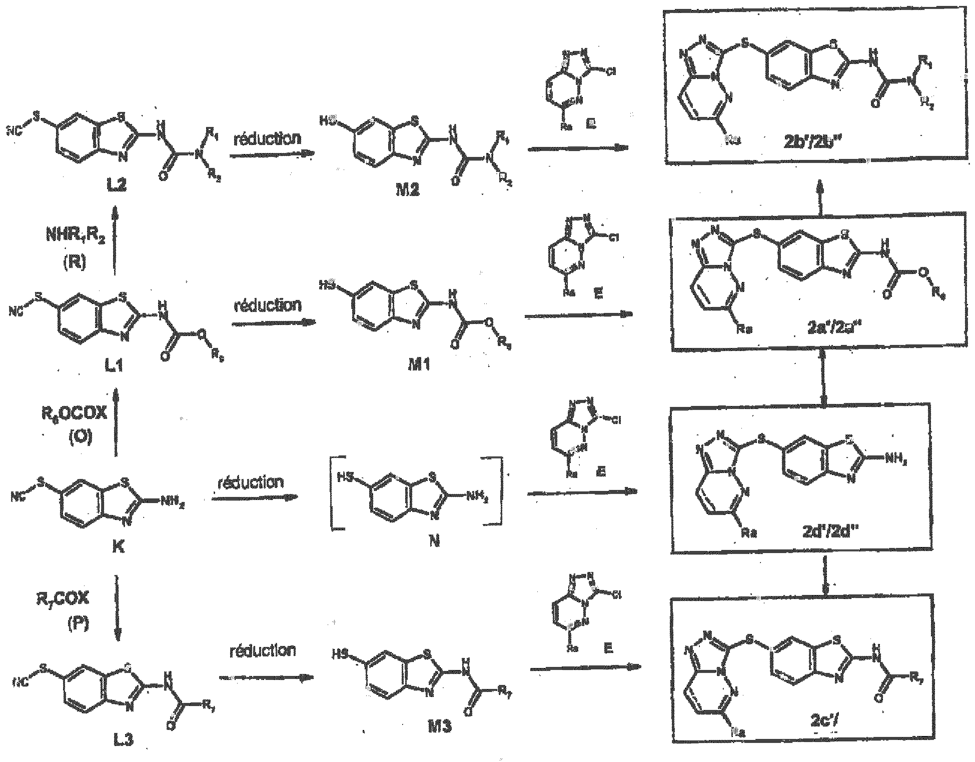 worin die Substituenten Ra, R1 und R2 die in Anspruch 1 angegebenen Bedeutungen besitzen, der Substituent R6 für einen Alkylrest, der gegebenenfalls **durch** eine NR3R4- oder OCH3-Gruppe substituiert ist, einen Phenylrest oder einen -(CH₂)n-Phenylrest steht, wobei n für eine ganze Zahl von 1 bis 2 steht, der Substituent R7 für einen Cycloalkyl- oder Alkylrest, der gegebenenfalls **durch** einen NR3R4- oder OCH3-Rest substituiert ist, steht, wobei R3 und R4 die in Anspruch 1 angegebenen Bedeutungen besitzen und n für eine ganze Zahl von 1 bis 2 steht;
in den Verbindungen der Formeln 2a'', 2b'', 2c'' und 2d'' ---- für eine Doppelbindung steht,
mit der Maßgabe, dass
- die Reduktion der Verbindungen der Formel (L1), (L2) bzw. (L3) zu den Verbindungen (M1), (M2) und (M3) mit DL-Dithiothreitol in Gegenwart von Natriumdihydrogencarbonat in einem Lösungsmittel und bei einer Temperatur in der Nähe von 80°C erhalten wird,
- die Reduktion der Verbindung K zu Verbindung (N) mit Natriumborhydrid in einem Lösungsmittel, in Gegenwart einer Base und bei einer Temperatur in der Nähe von 95°C oder zwischen 20°C und 95°C erhalten wird,
- mit der Maßgabe, dass man so **durch** die oben angegebenen Verfahren erhaltene Zwischenprodukte oder Produkte der Formel (I''b) gewüngchtenfalls und nötigenfalls zum Erhalt von anderen Zwischenprodukten oder anderen Produkten der Formel (I''b) einer oder mehreren dem Fachmann bekannten Transformationsreaktionen unterwirft, insbesondere:
a) einer Reaktion zur Veresterung einer Säurefunktion,
b) einer Reaktion zur Verseifung einer Esterfunktion zu einer Säurefunktion,
c) einer Reaktion zur Reduktion der freien oder veresterten Carboxyfunktion zu einer Alkoholfunktion,
d) einer Reaktion zur Transformation einer Alkoxyfunktion in eine Hydroxylfunktion oder auch einer Hydroxylfunktion in eine Alkoxyfunktion,
e) einer Reaktion zur Abspaltung von Schutzgruppen, die die geschützten reaktiven Gruppen tragen können,
f) einer Reaktion zur Versalzung mit einer anorganischen oder organischen Säure oder einer Base zum Erhalt des entsprechenden Salzes,
g) einer Reaktion zur Aufspaltung der racemischen Formen zu aufgespaltenen Produkten,
wobei die so erhaltenen Produkte der Formel (I''b) in allen möglichen racemischen, enantiomeren und diastereoisomeren isomeren Formen vorliegen.

4. Produkte der Formel (I''b) nach einem der Ansprüche 1 und 2 sowie die Additionssalze der Produkte der Formel (I''b) mit anorganischen und organischen Säuren oder mit anorganischen und organischen Basen, die pharmazeutisch unbedenklich sind, als Medikamente.

5. Pharmazeutische Zusammensetzungen, die als Wirstoff mindestens eines der Produkte der Formel (I''b) gemäß den Ansprüchen 1 und 2 oder ein pharmazeutisch unbedenkliche Salz dieses Produkts und einen pharmazeutisch unbedenklichen Träger enthalten.

6. Verwendung eines Produkts der Formel (I''b) gemäß einem der Ansprüche 1 und 2 zur Herstellung eines Medikaments zur Behandlung oder Prävention einer Erkrankung aus der folgenden Gruppe: Störungen der Blutgefäßproliferation, fibrotische Störungen, Störungen der Mesangialzellenproliferation, Stoffwechselstörungen, Allergien, Asthma, Thrombosen, Erkrankungen des Nervensystems, Retinopathie, Psoriasis, rheumatoide Arthritis, Diabetes, Muskeldegeneration und Krebserkrankungen.

7. Verwendung eines Produkts der Formel (I''b) gemäß einem der Ansprüche 1 und 2 zur Herstellung eines Medikaments zur Behandlung von Krebserkrankungen.

8. Verwendung nach Anspruch 7 zur Behandlung von soliden oder flüssigen Tumoren.

9. Verwendung nach Anspruch 7 oder 8 zur Behandlung von Krebserkrankungen, die gegenüber Zytotoxika resistent sind.

10. Verwendung nach einem der Ansprüche 7 bis 9 zur Behandlung von primären Tumoren und/oder Metastasen, insbesondere bei Krebserkrankungen des Magens, der Leber, der Niere, der Eierstöcke, des Kolons, der Prostata, der Lunge (NSCLC und SCLC), Glioblastomen, Krebserkrankungen der Schilddrüse, der Blase, des Bluts, bei Melanom, bei lymphoiden oder myeloiden hämopoetischen Tumoren, bei Sarkomen, bei Krebserkrankungen des Gehirns, des Kehlkopfs, des Lymphsystems, Krebserkrankungen der Knochen und der Bauchspeicheldrüse.

11. Verwendung eines Produkts der Formel (I''b) gemäß den Ansprüchen 1 und 2 zur Herstellung von Medikamenten für die Krebschemotherapie.

12. Verwendung eines Produkts der Formel (I''b) gemäß den Ansprüchen 1 und 2 zur Herstellung von Medikamenten für die Krebschemotherapie, die allein oder in Kombination verwendet werden.

13. Synthesezwischenprodukte der Formeln E', M1, M2 und M3 als neue technische Produkte: 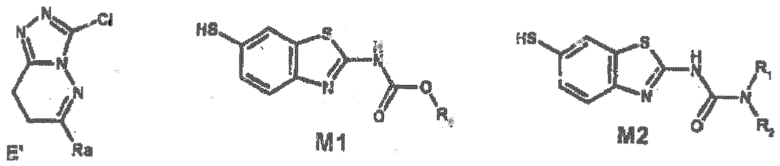 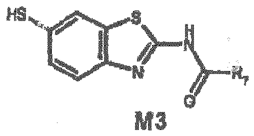 worin R6 für einen Alkylrest, der gegebenenfalls durch eine NR3R4- oder OCH3-Gruppe substituiert ist, einen Phenylrest oder einen -(CH₂)n-Phenylrest steht und n für eine ganze Zahl von 1 bis 2 steht; R7 für einen Cycloalkyl- oder Alkylrest, der gegebenenfalls durch einen NR3R4- oder OCH3-Rest substituiert ist, steht und Ra, R1, R2, R3 und R4 die in Anspruch 1 angegebenen Bedeutungen besitzen.

## Claims

1. Products of formula (I''b): 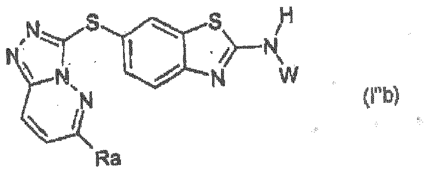 in which
Ra represents a hydrogen atom or else a chlorine atom or else the radical: 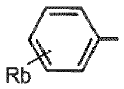 with Rb representing a halogen atom or an S-heteroaryl radical optionally substituted with a radical chosen from halogen atoms and the radicals: hydroxyl, alkyl and alkoxy containing from 1 to 4 carbon atoms, NH₂, NHalk and N(alk)₂;
W represents a hydrogen atom or an alkyl radical or the COR radical in which R represents:
- an alkyl radical optionally substituted with OCH₃ or NR3R4;
- a cycloalkyl radical;
- an alkoxy radical optionally substituted with OCH₃ or NR3R4, i.e. an O-(CH₂)ₙ-OCH₃ radical or an O-(CH₂)ₙ-NR3R4 radical; an O-phenyl radical or an O-(CH₂)ₙ-phenyl radical, with n representing an integer from 1 to 2;
- or the NR1R2 radical, in which R1 and R2 are such that one of R1 and R2 represents a hydrogen atom, a cycloalkyl radical, or an alkyl radical and the other of R1 and R2 represents an alkyl radical optionally substituted with NR3R4, or else R1 and R2 form with the nitrogen atom to which they are attached, a cyclic radical containing from 3 to 10 members and optionally containing one or more other heteroatoms chosen from O, S, N and NH;
with NR3R4 such that R3 and R4, which may be identical or different, represent a hydrogen atom or an alkyl radical, or else R3 and R4 form, with the nitrogen atom to which they are attached, a cyclic radical optionally containing one or more other heteroatoms chosen from O, S, N and NH, this radical, including the possible NH that it contains, being optionally substituted with one or more radicals chosen from alkyl radicals;
with the term "alkyl radical" or "Alk" denoting an alkyl radical containing from 1 to 6 carbon atoms;
with the term "cycloalkyl radical" denoting a saturated carbocyclic radical containing from 3 to 10 carbon atoms;
with the term "alkoxy" denoting a radical containing from 1 to 4 carbon atoms;
with the term "heteroaryl" denoting a monocyclic or bicyclic radical containing from 5 to 12 members;
said products of formula (I"b) being in any of the possible racemic, enantiomeric and diastereoisomeric isomer forms, and also the addition salts with mineral and organic acids or with mineral and organic bases of said products of formula (I"b).

2. Products of formula (I"b) as defined in Claim 1, corresponding to the following formulae:
- methyl (6-{[6-(4-fluorophenyl)[1,2,4]triazolo[4,3-b]pyridazin-3-yl]sulphanyl}-1,3-benzothiazol-2-yl)carbamate
- 1,1-dimethylethyl(6-{[6-(4-fluorophenyl)[1,2,4]triazolo[4,3-b]pyridazin-3-yl]sulphanyl}-1,3-benzothiazol-2-yl)carbamate
- 6-{[6-(4-fluorophenyl)[1,2,4]triazolo[4,3-b]pyridazin-3-yl]sulphanyl}-1,3-benzothiazol-2-amine
- 1-(2-morpholin-4-ylethyl)-3-[6-([1,2,4]triazolo[4,3-b]pyridazin-3-ylsulphanyl)-1,3-benzothiazol-2-yl]urea
- 1-(6-{[6-(4-fluorophenyl)[1,2,4]triazolo[4,3-b]pyridazin-3-yl]sulphanyl}-1,3-benzothiazol-2-yl)-3-(2-morpholin-4-ylethyl)urea
- 1-{2-[(2R,6S)-2,6-dimethylmorpholin-4-yl]ethyl}-3-(6-{[6-(4-fluorophenyl)-[1,2,4]triazolo[4,3-b]pyridazin-3-yl]sulphanyl}-1,3-benzothiazol-2-yl)urea
- (6-{[6-(4-fluorophenyl)[1,2,4]triazolo[4,3-b]pyridazin-3-yl]sulphanyl}-1,3-benzothiazol-2-yl)carbamate of 2-morpholin-4-ylethyl
- 1-(6-{[6-(4-fluorophenyl)[1,2,4]triazolo[4,3-b]pyridazin-3-yl]sulphanyl}-1,3-benzothiazol-2-yl)-3-[2-(4-methylpiperazin-1-yl)ethyl]urea
- N-(6-{[6-(4-fluorophenyl)[1,2,4]triazolo[4,3-b]pyridazin-3-yl]sulphanyl}-1,3-benzothiazol-2-yl)-4-morpholin-4-ylbutanamide
- 1-[2-(diethylamino)ethyl]-3-(6-{[6-(4-fluorophenyl)[1,2,4]triazolo[4,3-b]pyridazin-3-yl]sulphanyl}-1,3-benzothiazol-2-yl)urea
- phenyl 6-{[6-(4-fluorophenyl)[1,2,4]triazolo[4,3-b]pyridazin-3-yl]sulphanyl}-1,3-benzothiazol-2-yl)carbamate
- N-(6-{[6-(4-fluorophenyl)[1,2,4]triazolo[4,3-b]pyridazin-3-yl]sulphanyl}-1,3-benzothiazol-2-yl)acetamide
- 6-{[6-(3-fluorophenyl)[1,2,4]triazolo[4,3-b]pyridazin-3-yl]sulphanyl}-1,3-benzothiazol-2-amine
- N-(6-{[6-(4-fluorophenyl)[1,2,4]triazolo[4,3-b]pyridazin-3-yl]sulphanyl}-1,3-benzothiazol-2-yl)morpholine-4-carboxamide
- 6-{[6-(2-fluorophenyl)[1,2,4]triazolo[4,3-b]pyridazin-3-yl]sulphanyl}-1,3-benzothiazol-2-amine
- N-(6-{[6-(4-fluorophenyl)[1-,2,4]triazolo[4,3-b]pyridazin-3-yl]sulphanyl}-1,3-benzothiazol-2-yl)cyclopropanecarboxamide
- 1-(6{[6-(3-fluorophenyl)[1,2,4]triazolo[4,3-b]pyridazin-3-yl]sulphanyl}-1,3-benzothiazol-2-yl)-3-[2-(morpholin-4-yl)ethyl]urea
- N-(6-{[6-(3-fluorophenyl)[1,2,4]triazolo[4,3-b]pyridazin-3-yl]sulphanyl}-1,3-benzothiazol-2-yl)acetamide
- N-(6-{[6-(4-fluorophenyl)[1,2,4]triazolo[4,3-b]yridazin-3-yl]sulphanyl}-1,3-benzothiazol-2-yl)-2-methylpropanamide
- N-(6-{[6-(3-fluorophenyl)[1,2,4]triazolo[4,3-b]pyridazin-3-yl]sulphanyl}-1,3-benzothiazol-2-yl)cyclopropanecarboxamide
and also the addition salts with pharmaceutically acceptable mineral and organic adds or with pharmaceutically acceptable mineral and organic bases of said products of formula (I"b).

3. Process for preparing the products of formula (I"b) as defined in Claim 1, **characterized by** the following scheme: 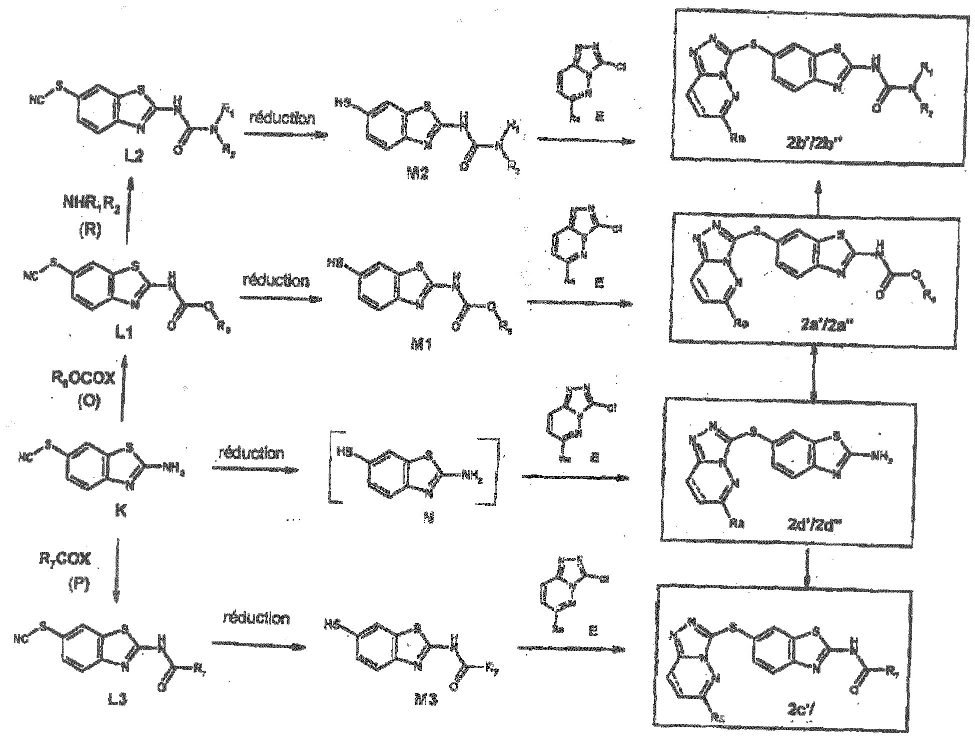 in which the substituents Ra, R1 and R2 have the meanings indicated in Claim 1, the substituent R6 represents an alkyl radical optionally substituted with an NR3R4 or OCH₃ group, a phenyl radical or a -(CH₂)ₙ-phenyl radical, and n represents an integer from 1 to 2, the substituent R7 represents a cycloalkyl or alkyl radical optionally substituted with an NR3R4 or OCH₃ radical, in which R3 and R4 have the meanings indicated in Claim 1 and n represents an integer from 1 to 2;
in the compounds of formulae 2a", 2b", 2c" and 2d", represents a double bond,
it being understood that
- the reduction of the compounds of formulae (L1), (L2) and (L3) respectively to give compounds (M1), (M2) and (M3) is obtained with DL-dithiothreitol, in the presence of sodium dihydrogen carbonate, in a solvent and at a temperature in the region of 80°C,
- the reduction of the compound K to give compound (N) is obtained with sodium borohydride in a solvent, in the presence of a base and at a temperature in the region of 95°C or between 20°C and 95°C,
it being understood that it is possible, if desired and if necessary, to subject intermediate products or products of formula (I"b) thus obtained by the processes indicated above, in order to obtain other intermediates or other products of formula (I"b), to one or more conversion reactions known to those skilled in the art, in particular:
a) a reaction for esterification of an acid function,
b) a reaction for saponification of an ester function to an acid function,
c) a reaction for reducing a free or esterified carboxyl function to an alcohol function,
d) a reaction for conversion of an alkoxy function to a hydroxyl function, or alternatively of a hydroxyl function to an alkoxy function,
e) a reaction for removal of the protecting groups that may be borne by the protected reactive functions,
f) a reaction for salification with a mineral or organic acid or with a base so as to obtain the corresponding salt,
g) a reaction for resolution of the racemic forms to resolved products,
said products of formula (I"b) thus obtained being in any of the possible racemic, enantiomeric and diastereoisomeric isomer forms.

4. As medicaments, the products of formula (I"b) as defined in either of Claims 1 and 2, and also the addition salts with pharmaceutically acceptable mineral and organic acids or with pharmaceutically acceptable mineral and organic bases of said products of formula (I"b).

5. Pharmaceutical compositions containing, as active ingredient, at least one of the products of formula (I"b) as defined in either of Claims 1 and 2, or a pharmaceutically acceptable salt of this product, and a pharmaceutically acceptable carrier.

6. Use of a product of formula (I"b) as defined in either of Claims 1 and 2, for the preparation of a medicament for use in the treatment or prevention of a disease chosen from the following group: blood vessel proliferation disorders, fibrotic disorders, 'mesangial' cell proliferation disorders, metabolic disorders, allergies, asthma, thrombosis, nervous system diseases, retinopathy, psoriasis, rheumatoid arthritis, diabetes, muscle degeneration and cancers.

7. Use of a product of formula (I"b) as defined in either of Claims 1 and 2, for the preparation of a medicament for use in the treatment of cancers.

8. Use according to Claim 7, for the treatment of solid or liquid tumours.

9. Use according to Claim 7 or 8, for the treatment of cancers resistant to cytotoxic agents.

10. Use according to one or more of Claims 7 to 9, for the treatment of primary tumours and/or metastases, in particular in gastric, hepatic, renal, ovarian, colon, prostate and lung (NSCLC and SCLC) cancers, glioblastomas, thyroid, bladder or breast cancers, in melanomas, in lymphoid or myeloid hematopoietic tumours, in sarcomas, in brain, larynx or lymphatic system cancers, bone cancers and pancreatic cancers.

11. Use of the products of formula (I"b) as defined in Claims 1 and 2, for the preparation of medicaments for use in cancer chemotherapy.

12. Use of the products of formula (I"b) as defined in Claims 1 and 2, for the preparation of medicaments for use in cancer chemotherapy alone or in combination.

13. As new industrial products, the synthesis intermediates of formulae E', M1, M2 and M3: 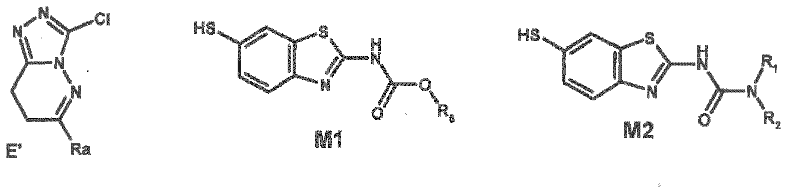 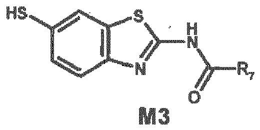 in which R6 represents an alkyl radical optionally substituted with an NR3R4 or OCH₃ group, a phenyl radical or a -(CH₂)ₙ-phenyl radical, and n represents an integer from 1 to 2; R7 represents a cycloalkyl or alkyl radical optionally substituted with an NR3R4 or OCH₃ radical; and Ra, R1, R2, R3 and R4 have the meanings indicated in Claim 1.
